# EUROPEAN PATENT SPECIFICATION

(11) **EP 3 611 270 B1**
(45) Date of publication and mention of the grant of the patent: **01.12.2021**
(21) Application number: 19171881.6
(22) Date of filing: 19.05.2010
(51) Int. Cl.: C12Q 1/00, G01N 33/50, A61K 39/00, C08G 83/00, A61K 48/00, C12N 15/87, C07K 14/00, G01N 33/543

(54) **KITS FOR IN VITRO ANTIGEN PRESENTATION, ASSESSING VACCINE EFFICACY, AND ASSESSING IMMUNOTOXICITY OF BIOLOGICS AND DRUGS**
KITS ZUR IN-VITRO-ANTIGEN-DARSTELLUNG, ZUR ÜBERPRÜFUNG DER WIRKSAMKEIT EINES IMPFSTOFFS UND ZUR ÜBERPRÜFUNG DER IMMUNTOXIZITÄT VON BIOLOGISCHEN STOFFEN UND ARZNEIMITTELN
KITS DE PRÉSENTATION D'ANTIGÈNE IN VITRO, D'ÉVALUATION DE L'EFFICACITÉ D'UN VACCIN, ET D'ÉVALUATION DE L'IMMUNOTOXICITÉ D'AGENTS BIOLOGIQUES ET DE MÉDICAMENTS

(30) Priority: 19.05.2009 US 17961409 P
(43) Date of publication of application: 19.02.2020
(62) Divisional of application: 10778304.5
(73) Proprietor: University Of Miami, Miami, Florida 33136 (US)
(72) Inventor: Daftarian, Pirouz, Mohammad, Miami, FL 33157 (US); Serafini, Paolo, Miami Shores, FL 33138 (US); Lemmon, Vance, Paul, Miami, FL 33133 (US); Kaifer, Angel, Coral Gables, FL 33134 (US); Blomberg, Bonnie, Beth, Coral Gables, FL 33134 (US); Chowdhury, Raquibul, Miami, FL 33179 (US); Kenyon, Norma, Miami, FL 33156 (US)
(74) Representative: Ferreccio, Rinaldo

(56) References cited:
- WO-A1-00/17639
- WO-A2-2010/115046
- US-A1- 2005 208 120
- AGADJANYAN M G ET AL: "PROTOTYPE ALZHEIMER'S DISEASE VACCINE USING THE IMMUNODOMINANT B CELL EPITOPE FROM BETA-AMYLOID AND PROMISCUOUS T CELL EPITOPE PAN HLA DR-BINDING PEPTIDE", THE JOURNAL OF IMMUNOLOGY, THE AMERICAN ASSOCIATION OF IMMUNOLOGISTS, US, vol. 174, no. 3, 1 February 2005 (2005-02-01), pages 1580-1586, XP008052934, ISSN: 0022-1767
- PETR NIEDERHAFNER ET AL: "Glycopeptide dendrimers, Part III-a review: Use of glycopeptide dendrimers in immunotherapy and diagnosis of cancer and viral diseases", JOURNAL OF PEPTIDE SCIENCE, vol. 14, no. 5, May 2008 (2008-05), pages 556-587, XP055055284, ISSN: 1075-2617, DOI: 10.1002/psc.1011
- PETER M. H. HEEGAARD ET AL: "Dendrimers for Vaccine and Immunostimulatory Uses. A Review", BIOCONJUGATE CHEMISTRY, vol. 21, no. 3, 4 November 2009 (2009-11-04), pages 405-418, XP055055308, ISSN: 1043-1802, DOI: 10.1021/bc900290d
- P. DAFTARIAN ET AL: "Peptide-Conjugated PAMAM Dendrimer as a Universal DNA Vaccine Platform to Target Antigen-Presenting Cells", CANCER RESEARCH, vol. 71, no. 24, 15 December 2011 (2011-12-15), pages 7452-7462, XP055055287, ISSN: 0008-5472, DOI: 10.1158/0008-5472.CAN-11-1766

## Description

### FIELD OF THE INVENTION

The invention relates generally to the fields of chemistry, diagnostics, and immunology. More particularly, the invention relates to nanoparticle-based kits for assessing efficacy of a vaccine.

### BACKGROUND

Unlike monitoring antibody responses, immunomonitoring of T cells (e.g. upon vaccination) currently is inaccurate, does not correlate with the efficacy of vaccines and other interventions, requires costly artificial cocktails of peptides (uncertain, MHC-restricted and incomplete cocktails of peptides) and does not provide much assistance for making the right decisions to move forward to Phase II or III clinical trials, for example, as they are sometimes misleading. Evaluation of cellular immune responses against specific antigens requires the expression of antigens on autologous antigen-presenting-cells (APCs) associated with major histocompatibility complex (MHC) molecules in order to elicit appropriate T cell-mediated responses. The interaction of presented antigens with specific T cell clono-types results in induction of cytokines, proliferation, and/or lysis of "self' target cells that express the specific antigen tagged by MHC molecules. The MHC genomic region is present in all APCs. MHC is an essential component of the immune system that plays important roles in immune responses to pathogens, tumor antigens as well as in autoimmunity. The proteins encoded by the MHC genes are expressed on the surface of cells that present both self antigens, from the cell itself, and nonself antigen fragments from pathogens or tumor cells to various T cells enabling them to i) provide help for initiation of immune responses, ii) help T cells kill invading pathogens/tumor cells/cells-infected with pathogens, and iii) coordinate the maturation of antibodies against nonself antigens. Class II MHC molecules are expressed largely on specialized APCs such as macrophages, monocytes, dendritic cells and B cells and are recognized by helper T lymphocytes. This in turn induces proliferation of helper T lymphocytes and amplification of the MHC-antigen specific immune response. The level of activation and proliferation of the helper T cells is proportional to the intensity of immune responses and forms the basis for measurement of a cellular immune response to infection or therapeutic intervention such as vaccination or immunotherapy. For example, immunomonitoring of cellular immune responses upon any vaccination requires that such vaccine antigens be expressed on self APC while accompanied by self MHC molecules, also specific or unique to each individual. Immunodiagnostics of T cell responses are hampered by such MHC/human leukocyte antigen (HLA) restriction in that target cells (APCs) must have the same MHC/HLA as effector cells (T cells). T cells can only see the antigen in the context of their own MHC (syngeneic). Indeed, T cell epitopes of each and every antigen which are specific for each and every HLA haplotype must be identified. This, in fact, is a very difficult and costly process. A mixture of such epitopes that contains all possible epitopes for all various MHCs must be used to stimulate PBMCs for their T cell responses. The challenge is that only limited numbers of T-cell epitopes are identified and they do not correlate with the host T cell response *in vivo,* e.g. not only is there poor correlation between such responses and the efficacy of a vaccine, such techniques cannot predict the immune system's adverse reaction to a biologic. Alternative methods are hampered, for example, because antigen uptake by APCs is so poor. Unfortunately, APC uptake of proteins, antigens and DNA plasmids is pitiably weak and there is no simple method to transfect self APCs.

To prepare targets for measurement of cellular immune responses, investigators have attempted EBV infection or transfection of immortalized autologous B cells, as well as stimulation of peripheral blood mononuclear cells (PBMCs) by CpG, or co-culturing them with cells expressing CD40-ligand followed by transfection with vectors expressing vaccine antigens. Alternatively, overlapping peptide arrays, or a cocktail of selected known peptides from the vaccine antigens, have been used to target self APCs. Alternatively, tetramers (that include epitopes) tagged with fluorochromes are used to bind to specific T cells to quantify them. Use of peptides, however, has several limitations, including: i) limited to linear peptides, ii) limited to only known epitopes of antigens, and iii) specificity for either MHC class I or II presentation based on size. These methods can only be used on a small scale and in specialized laboratories, and they are expensive, complicated, and difficult to validate and standardize.

Current methods for evaluation of an immune response to infection or immunization are limited by the lack of accurate, rapid and simple immunomonitoring methods of cellular immune responses. Currently, the objective response rate in clinical studies is rarely >10%, preventing meaningful correlations of T-cell response rates with clinical responses. Accurate measurement of the immune response is an indicator of success of a therapeutic or prophylactic intervention and is of paramount importance in evaluation of vaccine efficacy. Major limitations of current methods include i) a lack of consensus on the method of choice, ii) poor reproducibility, iii) a requirement for specialized skills and instrumentation, iv) high costs, and v) a low correlation with protection. Of significant concern is that the antigen used for binding to a specific antibody, or processing by APCs to interrogate cellular immune responses, may not be an accurate representation of forms seen *in vivo* during infection, limiting the ability of these assays to provide an accurate picture of humoral or cellular immunity in an individual. Moreover, approaches which utilize a cocktail of peptides (epitopes) may be inappropriately targeted, since in most cases they only present a limited number of known linear epitopes that are limited by MHC restriction. The use of recombinant, subunit antigen is not an option since APCs do not uptake such antigens (or do so poorly) and they may not be representative of native antigen configurations, particularly as processed by APCs. These approaches, therefore, greatly restrict the accuracy of measurements of cellular immune responses, and limit the usefulness of these assays in predicting clinical efficacy.

Currently, there are no effective methods or reagents for evaluating the cellular immune response after vaccination or in response to a drug or biologic. There is thus a significant need for methods and reagents for accurately predicting clinical efficacy of a vaccine, drug or biologic that can be used on a large scale in any clinical setting and that are easy to produce.

### SUMMARY

The present invention concerns a kit for assessing efficacy of a vaccine, the kit comprising: a) a plurality of charged highly branched polymeric dendrimers each having conjugated thereto at least one Pan-HLA-DR-binding epitope (PADRE) and at least one agent selected from the group consisting of: peptide or polypeptide antigen, nucleic acid encoding the at least one antigen, negative control polypeptide, and nucleic acid encoding a control polypeptide, wherein the at least one PADRE and the at least one agent are conjugated to the exterior surface of the plurality of charged highly branched polymeric dendrimers such that the at least one PADRE specifically binds to professional antigen presenting cells; b) at least one buffer; c) a solid substrate; and e) instructions for use.

Moreover, described herein are nanoparticle-based compositions, assays, kits, methods and platforms for delivering an antigen (peptides, proteins) or a nucleic acid encoding an antigen to professional APCs (PAPCs) that result in the generation of autologous APCs that present a natural peptide repertoire of the antigen for use in assessing the efficacy of a vaccine (e.g., a cytotoxic T lymphocyte (CTL) response to a particular antigen) or other therapy or intervention (cell-based therapy, adjuvant therapy, etc.). The compositions, kits, assays and methods also can be used for delivering a drug or biologic or portion thereof to APCs for assessing the immunogenicity of drugs and biologics. The composition, kits, assays and methods involve the combined use of MHC targeting, universal DR binding peptides (e.g., PADRE OR INFLUENZA HA T HELPER EPITOPE: SFERFEIFPKEC (SEQ ID NO:28), HA) with charged (e.g., positively-charged) highly branched polymeric dendrimers (e.g., PAMAM and other dendrimers) as vehicles for the targeted delivery of nucleic acids, peptides, biologics, drugs, or polypeptides to APCs, giving rise to a new nanoparticle-based method for assessing the immune response (e.g., CTL response, B cell response) to a vaccination or other therapy or intervention, or for assessing the immunogenicity of a biologic or drug. Targeted delivery of nucleic acids, peptides, biologics, drugs, or polypeptides to APCs for effective expression and processing generates more physiologically relevant target antigens for evaluation of cell-mediated immune responses to vaccination, for example, and provides a low-cost approach for rapid generation of reagents and development of assay systems for more accurate profiling of immunological responses to infection, immunization, and other therapies or interventions. Immunoevaluation kits using targeted nanoparticle-based antigen delivery are described herein.

A typical composition described herein for assessing the efficacy of a vaccine or other therapy or intervention or assessing the immunogenicity of a drug or biologic includes a charged (e.g., positively-charged) highly branched polymeric dendrimer conjugated to an MHC targeting and universal DR binding peptide (e.g., an epitope such as the tetanus toxin 582-599, the PADRE or Influenza HA T helper epitope: SFERFEIFPKEC (SEQ ID NO:28)), at least one polypeptide antigen or a nucleic acid encoding the at least one antigen, and optionally Poly I-C. The positively-charged highly branched polymeric dendrimers described herein effectively bind negatively-charged biomolecules including DNA, RNA and others. Charged (e.g., positively-charged) highly branched polymeric dendrimers conjugated to a universal DR binding peptide (e.g., an epitope such as the PADRE or Influenza HA T helper epitope: SFERFEIFPKEC (SEQ ID NO:28)) provide for specific antigen delivery to PAPCs. The kits, assays, methods and compositions described herein encompass all MHC class II binding peptides, and provide for specific and efficient transfection of PAPCs, and a universal assay for evaluating the efficacy of any vaccine or other therapy or intervention as well as evaluating the immunogenicity of a drug, allergen, or biologic.

Antigens or nucleic acids encoding the antigens are complexed with a peptide-derivatized-dendrimer (referred to herein as "PDD") where the peptide(s) is (are) a universal DR binding peptide(s) (e.g., a T helper epitope(s)) that binds MHC class II in the majority of humans. The complex of universal DR binding peptide-(e.g., amino acids 582-599 of tetanus toxin, PADRE, etc.)-derivatized-dendrimer and antigen (or DNA or RNA encoding the antigen(s)) are used to deliver such cargoes into cells in a way that they process and present the antigen specifically in the APCs in PBMC preparations, and convert them to antigen-expressing autologous APCs (referred to herein as "target cells"). They are thus particularly useful for determining if a subject who has received a therapy or intervention (e.g., vaccination) for treating or preventing a pathology (e.g., infection) has mounted an immune response to the therapy or intervention as well as quantitating the immune response. If the subject has mounted an immune response to the therapy or intervention, the subject will have reacting, primed (sensitized) T cells that are specific for the therapy or intervention. For example, if a subject receives a vaccination for influenza, the vaccine containing at least one influenza antigen, the subject will develop reacting, primed (sensitized) T cells that are specific for the influenza antigen if the vaccine was successful in promoting an immune response against the influenza antigen in the subject. Determining if a subject has reacting, primed (sensitized) T cells that are specific for the therapy or intervention typically involves examining one or more samples from the subject for levels of cytokines (e.g., IFN-γ), growth factors, cell markers, enzymes, chemokines or any other molecule or marker that is indicative of an immune response to a particular therapy or intervention. To correlate a specific immune response with the efficacy of a vaccine or other therapy or intervention, any suitable assay that measures T helper cell or B cell activation and proliferation and/or levels and expression of one or more molecules or markers (e.g., cytokines) that is indicative of an immune response to the vaccine or other therapy or intervention can be used. Examples of such assays include CTL and cytokine assays. Samples that are obtained from a subject for analyzing levels of cytokines (e.g., IFN-γ, interleukins, chemokines), growth factors, cell markers, enzymes, chemokines or any other molecule or marker that is indicative of an immune response to a particular therapy or intervention, generally include PBMCs, blood, splenocytes, or lymph node cells. A therapy or intervention as described herein includes, for example, any adjuvant therapy, any immunotherapies to enhance or reduce immune responses, any cell-based therapies, etc.

The specific delivery of antigen or nucleic acid encoding antigen to APCs for assessing the efficacy of a vaccine or other therapy or intervention as described herein results in the mimicking of native antigen presentation and allows more accurate and relevant measurements of mammalian (e.g., human) immune responses to antigens, infections, immunizations, and other therapies and interventions. A universal DR binding peptide-derivatized dendrimer complexed with antigens or nucleic acid (e.g., plasmid) encoding for an antigen as described herein specifically targets APCs, and converts these into APCs that present the antigen (referred to herein as target cells). One of the advantages of the compositions, kits, methods and assays described herein is based on the fact that an antigen-specific immune response can be evaluated accurately only when the antigen is presented in its native configuration. Unlike antibody responses, immunomonitoring of T cells (e.g. upon vaccination), currently, is not quite accurate, it does not correlate with the efficacy of vaccines and other interventions, and it requires costly, uncertain, and incomplete MHC-restricted artificial cocktails of peptides. Current methods are not useful for making the right decisions to move forward to Phase II or III trials with a particular drug or biologic, as they are sometimes misleading, contributing to the failure of many highly costly clinical trials. Current assays measure CTL responses via *in vitro* assays in which immune responses against related peptides, recombinant antigens, proteins or inactive viruses are tested. In contrast, the compositions, kits, assays and methods described herein include a universal class II specific -peptide (e.g., universal T helper epitopes such as SSVFNVVNSSIGLIM (SEQ ID NO:29) from *Plasmodium falciparum,* FNNFTVSFWLRVPKVSASHLE (SEQ ID NO:30) from Tetanus Toxoid or PADRE, a synthetic peptide, to list only a few examples) complexed with a dendrimer and an antigen or a nucleic acid encoding an antigen that when transfected into mammalian PBMCs, results in a broad and representative cellular response to the antigen if the host from whom the PBMCs are drawn had been previously exposed to the antigen (e.g., by vaccination). The specific delivery of antigen or plasmid DNA results in processing and presentation of antigen associated epitopes in the context of self MHC that should represent possible peptides or resemble a natural peptide repertoire derived from an antigen of interest. Such autologous APCs (from PBMCs) act as targets to evaluate effector (T cell) responses. Total cell-mediated immune responses can be evaluated using standard methods including, for example, an IFNγ ELISpot assay. The compositions, kits, assays and methods described herein provide a low-cost approach for rapid generation of reagents and more accurate profiling of immunological responses to infection, immunization, and other therapeutic interventions.

Unless otherwise defined, all technical terms used herein have the same meaning as commonly understood by one of ordinary skill in the art to which this invention belongs.

As used herein, a "nucleic acid" or a "nucleic acid molecule" means a chain of two or more nucleotides such as RNA (ribonucleic acid) and DNA (deoxyribonucleic acid), and chemically-modified nucleotides. A "purified" nucleic acid molecule is one that is substantially separated from other nucleic acid sequences in a cell or organism in which the nucleic acid naturally occurs (e.g., 30, 40, 50, 60, 70, 80, 90, 95, 96, 97, 98, 99, 100% free of contaminants). The terms include, e.g., a recombinant nucleic acid molecule incorporated into a vector, a plasmid, a virus, or a genome of a prokaryote or eukaryote. Examples of purified nucleic acids include cDNAs, fragments of genomic nucleic acids, nucleic acids produced polymerase chain reaction (PCR), nucleic acids formed by restriction enzyme treatment of genomic nucleic acids, recombinant nucleic acids, and chemically synthesized nucleic acid molecules. A "recombinant" nucleic acid molecule is one made by an artificial combination of two otherwise separated segments of sequence, e.g., by chemical synthesis or by the manipulation of isolated segments of nucleic acids by genetic engineering techniques.

When referring to an amino acid residue in a peptide, oligopeptide or protein, the terms "amino acid residue", "amino acid" and "residue" are used interchangably and, as used herein, mean an amino acid or amino acid mimetic joined covalently to at least one other amino acid or amino acid mimetic through an amide bond or amide bond mimetic.

As used herein, "protein" and "polypeptide" are used synonymously to mean any peptide-linked chain of amino acids, regardless of length or post-translational modification, e.g., glycosylation or phosphorylation.

When referring to a nucleic acid molecule, polypeptide, or infectious pathogen, the term "native" refers to a naturally-occurring (e.g., a wild-type (WT)) nucleic acid, polypeptide, or infectious pathogen.

As used herein, the term "antigen" or "immunogen" means a molecule that is specifically recognized and bound by an antibody.

When referring to an epitope (e.g., T helper epitope), by biological activity is meant the ability to bind an appropriate MHC molecule.

The terms "specific binding" and "specifically binds" refer to that binding which occurs between such paired species as enzyme/substrate, receptor/agonist, antibody/antigen, etc., and which may be mediated by covalent or non-covalent interactions or a combination of covalent and non-covalent interactions. When the interaction of the two species produces a noncovalently bound complex, the binding which occurs is typically electrostatic, hydrogenbonding, or the result of lipophilic interactions. Accordingly, "specific binding" occurs between a paired species where there is interaction between the two which produces a bound complex having the characteristics of an antibody/antigen or enzyme/substrate interaction. In particular, the specific binding is characterized by the binding of one member of a pair to a particular species and to no other species within the family of compounds to which the corresponding member of the binding member belongs.

As used herein, the terms "Pan-DR epitopes," "Pan-HLA-DR-binding epitope," "PADRE" and "PADRE peptides" mean a peptide of between about 4 and about 20 residues that is capable of binding at least about 7 of the 12 most common DR alleles (DR1, 2w2b, 2w2a, 3, 4w4, 4w14, 5, 7, 52a, 52b, 52c, and 53) with high affinity. "High affinity" is defined herein as binding with an IC₅₀% of less than 200 nm. For example, high affinity binding includes binding with an IC₅₀% of less than 3100 nM. For binding to Class II MHC, a binding affinity threshold of 1,000 nm is typical, and a binding affinity of less than 100nm is generally considered high affinity binding. Construction and use of PADRE peptides is described in detail in U.S. Patent No. 5,736,142.

As used herein, the phrase "DR binding peptide" means a peptide that binds to MHC class II, e.g., a peptide that binds to human MHC class II.

By the phrase "universal DR binding peptide" is meant a peptide that binds to anywhere on MHC class II molecules, e.g., to a large number of MHC of humans and/or mice and/or non-human primates.

A "T helper peptide" as used herein refers to a peptide recognized by the T cell receptor of T helper cells. For example, the PADRE peptides described herein are T helper peptides. A T helper peptide is one example of a universal DR binding peptide.

As used herein, the term "dendrimer" means a charged (e.g., positively-charged, negatively-charged), highly branched polymeric macromolecule with roughly spherical shape. An example of a positively-charged, highly branched polymeric dendrimer is a PAMAM dendrimer. By the terms "PAMAM dendrimer" and "poly-amidoamine dendrimer" is meant a type of dendrimer in which tertiary amines are located at branching points and connections between structural layers are made by amide functional groups.

By the terms "PAMAM dendrimer" and "poly-amidoamine dendrimer" is meant a type of dendrimer in which tertiary amines are located at branching points and connections between structural layers are made by amide functional groups. PAMAM dendrimers exhibit many positive charges on their surfaces.

By the term "derivatized dendrimer" is meant a dendrimer having one or more functional groups conjugated to its surface.

A "universal DR binding peptide-derivatized dendrimer" is a nanoconstruct in which one or more universal DR binding peptides are covalently attached to the functional groups on the surface of a charged (e.g., positively-charged) highly branched polymeric dendrimer (e.g., a PAMAM dendrimer).

A "PADRE-derivatized dendrimer" or "PADRE-dendrimer" is a nanoconstruct in which one or more PADRE peptides are covalently attached to the functional groups on the surface of a charged (e.g., positively-charged) highly branched polymeric dendrimer (e.g., a PAMAM dendrimer).

By the term "conjugated" is meant when one molecule or agent is physically or chemically coupled or adhered to another molecule or agent. Examples of conjugation include covalent linkage and electrostatic complexation. The terms "complexed," "complexed with," and "conjugated" are used interchangeably herein.

As used herein, the phrase "sequence identity" means the percentage of identical subunits at corresponding positions in two sequences (e.g., nucleic acid sequences, amino acid sequences) when the two sequences are aligned to maximize subunit matching, i.e., taking into account gaps and insertions. Sequence identity can be measured using sequence analysis software (e.g., Sequence Analysis Software Package from Accelrys CGC, San Diego, CA).

The phrases "isolated" or biologically pure" refer to material which is substantially or essentially free from components which normally accompany it as found in its native state.

As used herein, the term "nanoparticle" means a microscopic particle whose size is measured in nanometers. For example, a nanoparticle is a PADRE-dendrimer conjugate or a particle combining several PADRE-dendrimer conjugates and nucleic acid or amino acid material with a total diameter in the range of approximately 2-500 nm.

The term "antibody" is meant to include polyclonal antibodies, monoclonal antibodies (mAbs), chimeric antibodies, humanized antibodies, anti-idiotypic (anti-Id) antibodies to antibodies that can be labeled in soluble or bound form, as well as fragments, regions or derivatives thereof, provided by any known technique, such as, but not limited to, enzymatic cleavage, peptide synthesis or recombinant techniques.

As used herein the term "adjuvant" means any material which modulates to enhance the humoral and/or cellular immune response.

As used herein, the terms "displayed" or "surface exposed" are considered to be synonyms, and refer to antigens or other molecules that are present (e.g., accessible to immune site recognition) at the external surface of a structure such as a nanoparticle (e.g., PADRE-dendrimer).

By the term "multivalent" is meant that more than one copy or type of antigen or molecule is displayed on a nanoparticle.

As used herein, "vaccine" includes all prophylactic and therapeutic vaccines.

As used herein, the term "biologic" refers to a wide range of medicinal products such as vaccines, blood and blood components, allergenics, somatic cells, genes expressing a product in gene therapy, tissues, and recombinant therapeutic proteins created by recombinant DNA technology, antibodies, synthetic drugs, and long peptides (polypeptides), synthetic compounds, and (glycol)proteins.

By the phrase "immune response" is meant induction of antibody and/or immune cell-mediated responses specific against an antigen or antigens or allergen(s) or drug or biologic. The induction of an immune response depends on many factors, including the immunogenic constitution of the challenged organism, the chemical composition and configuration of the antigen or allergen or drug or biologic, and the manner and period of administration of the antigen or allergen or drug or biologic. An immune response has many facets, some of which are exhibited by the cells of the immune system (e.g., B-lymphocytes, T-lymphocytes, macrophages, and plasma cells). Immune system cells may participate in the immune response through interaction with an antigen or allergen or other cells of the immune system, the release of cytokines and reactivity to those cytokines. Immune responses are generally divided into two main categories - humoral and cell-mediated. The humoral component of the immune response includes production of antibodies specific for an antigen or allergen or drug or biologic. The cell-mediated component includes the generation of delayed-type hypersensitivity and cytotoxic effector cells against the antigen or allergen.

As used herein, the term "treatment" is defined as the application or administration of a therapeutic agent to a patient, or application or administration of the therapeutic agent to an isolated tissue or cell line from a patient, who has a disease, a symptom of disease or a predisposition toward a disease, with the purpose to cure, heal, alleviate, relieve, alter, remedy, ameliorate, improve or affect the disease, the symptoms of disease, or the predisposition toward disease.

The terms "patient" "subject" and "individual" are used interchangeably herein, and mean a mammalian subject who is to be treated, who has been treated, or who is being considered for treatment, with human patients being preferred. In some cases, the methods, kits, compositions and assays described herein find use in experimental animals, in veterinary applications, and in the development of animal models for disease, including, but not limited to, rodents including mice, rats, and hamsters, as well as non-human primates.

Although compositions, kits, assays and methods similar or equivalent to those described herein can be used in the practice or testing of the present invention, suitable compositions, kits, assays and methods are described below. In the case of conflict, the present specification, including definitions, will control. The particular embodiments discussed below are illustrative only and not intended to be limiting.

### BRIEF DESCRIPTION OF THE DRAWINGS

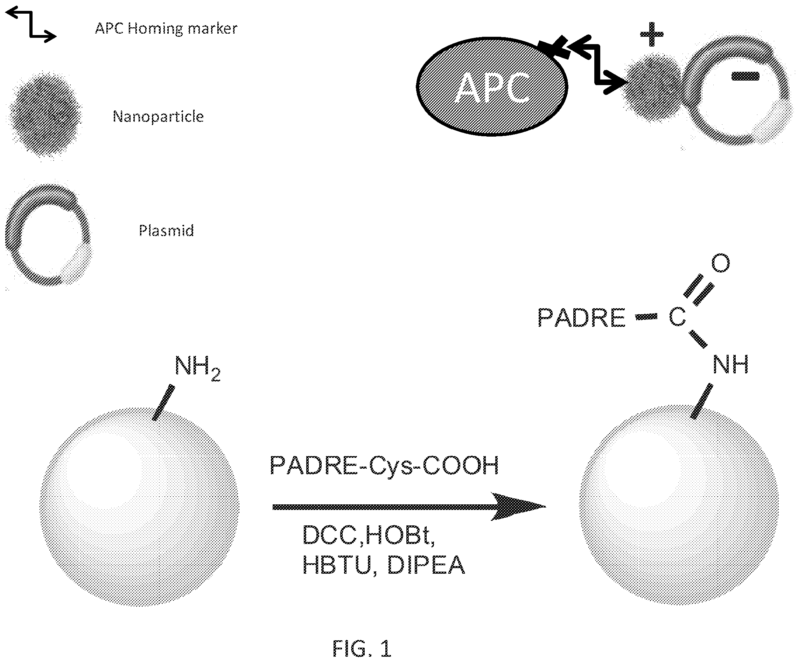

FIG. 1 is a pair of schematics showing the PADRE-dendrimer that may be mixed with plasmid or linked to a peptide or polypeptide antigen to target APCs. FIG. 1 illustrates that the PADRE-dendrimers described herein provide a platform in which any antigen of interest or nucleic acid encoding any antigen of interest can be incorporated. The PADRE-dendrimers described herein are taken up by professional APCs.

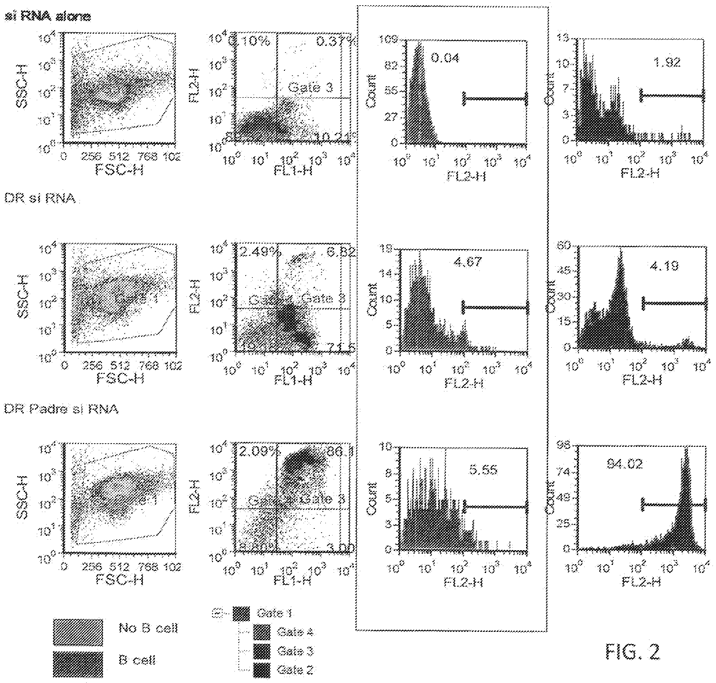

FIG. 2 is a series of dot plot flow cytometry images of analysis of human B cells showing *in vitro* delivery of PADRE-dendrimers complexed with a short nucleic acid sequence tagged with a red fluorochrome. This nucleic acid is a red-labeled dsRNA oligomer designed for use in RNAi analysis to facilitate assessment and optimization of dsRNA oligonucleotides delivery into mammalian cells. Cells were co-cultured with the PADRE-dendrimers/multinucleotide complexes or controls for 4 hours after which the media was removed and fresh media was added. The images show the delivery of dsRNA oligomer tagged with Alexa Fuor into purified Human B cells. The lowest image in the fourth column of images shows the delivery of the oligo in approximately 92% of cells.

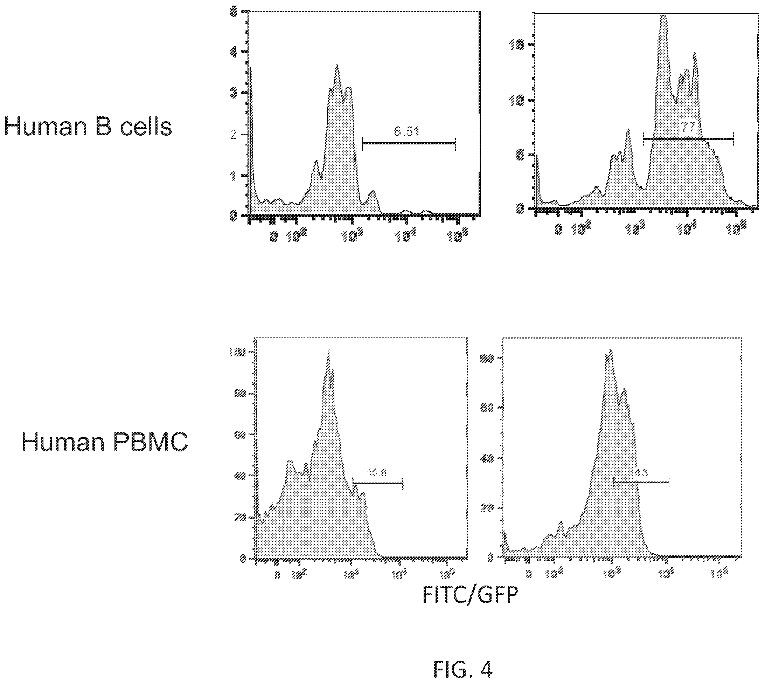

FIG. 4 is a series of flow cytometry histograms showing the expression of GFP in human peripheral blood mononuclear cells (PBMC), lower panel, and in human B cells, upper panel, upon co-culturing GFP plasmid (5 µg) complexed with Dendrimer-PADRE. Dendrimer/GFP-plasmid complex was used as a control, left histograms.

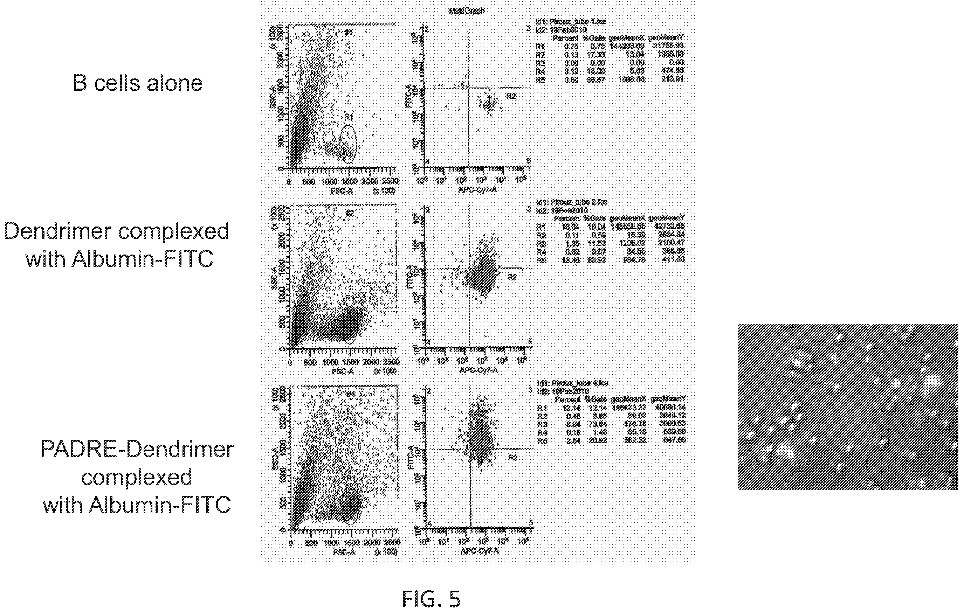

FIG. 5 is a series flow cytometry dot plots showing the *in vitro* delivery of a protein, Albumin-FITC, into human B cells by PDD. The left images show PDD/Albumin-FITC delivery into purified human B cells. Human purified B cells were collected and were co-cultured with PDD/Albumin-FITC. The left histograms show the delivery of Albumin-FITC in human B cells the morning after the PDD/Albumin-FITC added to human B cells. The Top histogram shows B cells alone, the histogram in the Middle shows the Dendrime/Albumin-FITC complex plus B cells and the lower histogram depicts the results of PDD/Albumin-FITC complex added to human B cells. The right picture is the image of fluorescent microscope of Albumin uptake by B cells one-hour post addition of PDD/Albumin-FITC complex.

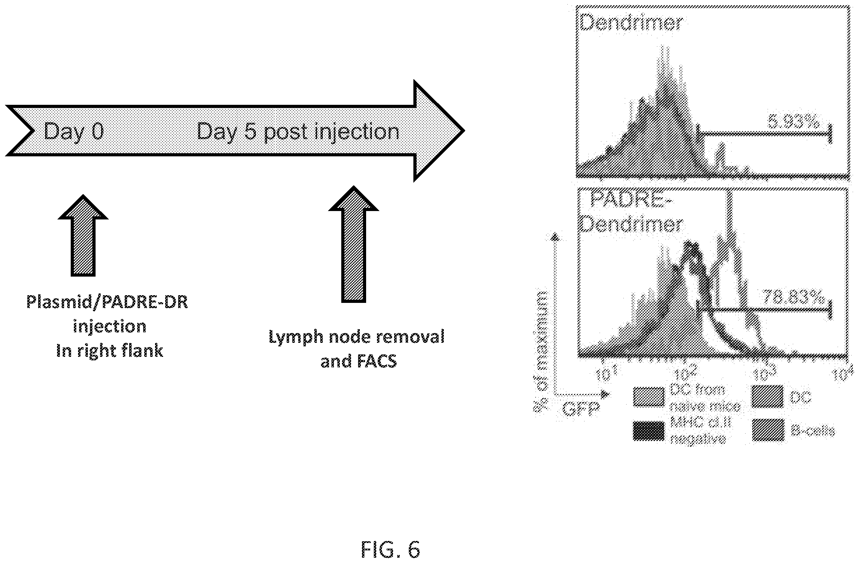

FIG. 6 is a series of flow cytometry dot plots showing the *in vivo* targeting of DCs in the lymph node. The left image depicts a schematic of a timeline for injection and lymph node removal and analysis and the right image shows a pair of flow cytometry dot plots upon analysis of data obtained from cells of the lymph node adjacent to PDD/GFP-plasmid or Dendrimer/GFP-plasmid injection site versus a naive lymph node. These images show the efficacy of *in vivo* PADRE-denhdrimer targeting of mouse DCs and B cells in an injection site neighboring the lymph node. Lymph cells were stained with CD11c (DC marker), MHC class II and CD20 (B cell marker). The histograms in the right top show that Dendrimer/GFP-plasmid injection resulted in the expression of GFP in approximately 6% of DCs while the lower dot plot clearly shows that PDD/GFP-plasmid injection resulted in the expression of GFP in > 70% of DCs.

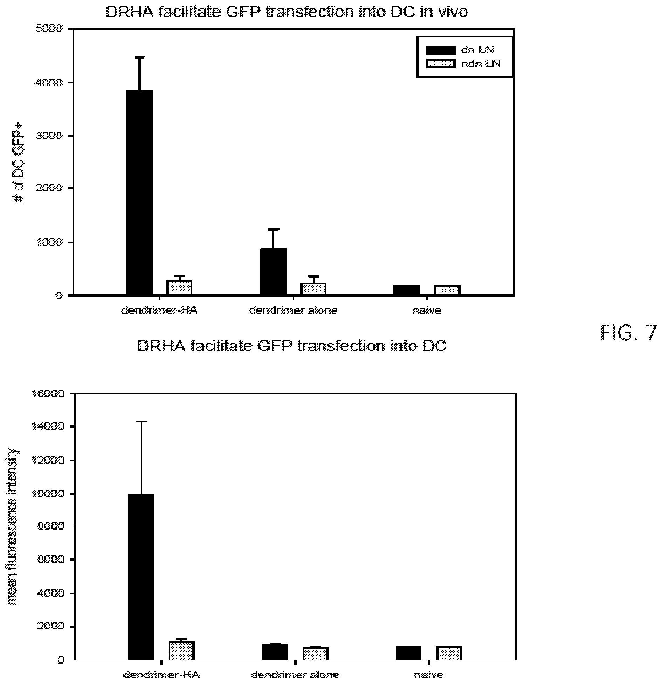

FIG. 7 is a pair of graphs showing that DRHA, a dendrimer decorated with a different T helper epitope, *in vivo* targeting DCs in the lymph node shows that DRHA facilitates GFP transfection into DCs. This experiment is similar to the one described in FIG.6 with the difference that Balb/c mice have been used in conjunction with dendrimer conjugated with Iadrestricted HA peptide. The lymph node adjacent to the DRHA/GFP-plasmid or Dendrimer/GFP-plasmid injection site and a naive lymph node were removed on day 5 post-injection of DRHA/GFP-plasmid or Dendrimer/GFP-plasmid. The charts show the results of the flow cytometry analysis of data obtained from cells of the lymph node after staining with CD11c (DC marker) for DC. The top pane shows the number of DC positive for GFP found draining lymph nodes of mice treated as indicated. The lower panel shows the mean fluorescence intensity of GFP within the DC. These results clearly indicate not only that DRHA augment the number of DC transfected *in vivo* but, also the number of plasmid molecules that get into the cells.

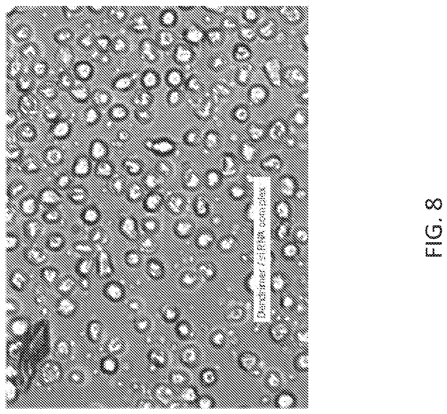

FIG. 8 is a micrograph of human B cells transfected with PADRE-dendrimer complexed with a red(Alexa Fluor)-labeled dsRNA oligomer oligo

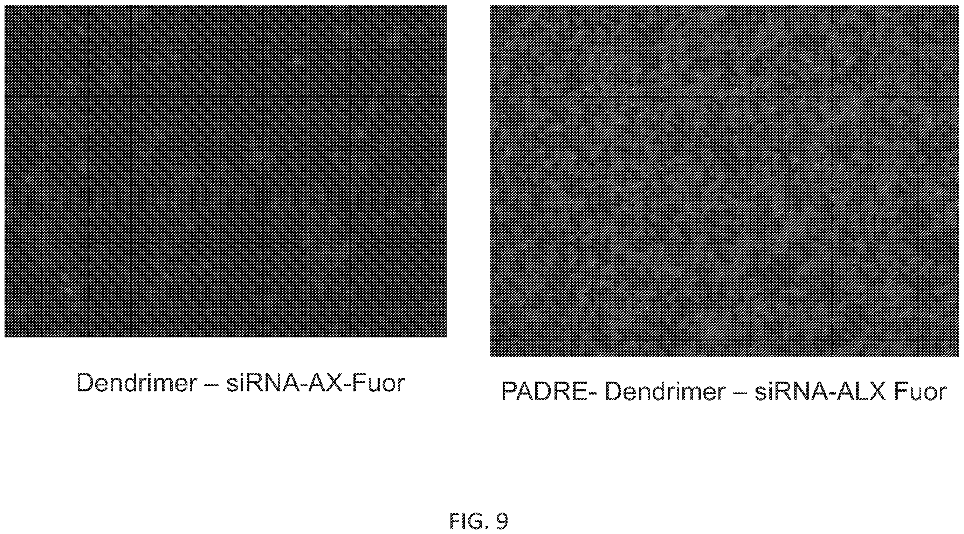

FIG. 9 is a pair of micrographs of PBMCS from Baboons transfected with dendrimer complexed with a red(Alexa Fluor)-labeled dsRNA oligomer (left panel) and cells transfected with PADRE-dendrimer complexed with a red(Alexa Fluor)-labeled dsRNA oligomer (right panel).The flurescent microscope images were taken two hours post addition of PDD/dsRNA-Alexa-Fluor or control complex to Baboon PBMC. The image shows high efficacy of targeted delivery of multinucleutides to PBMC of monkey via PDD.

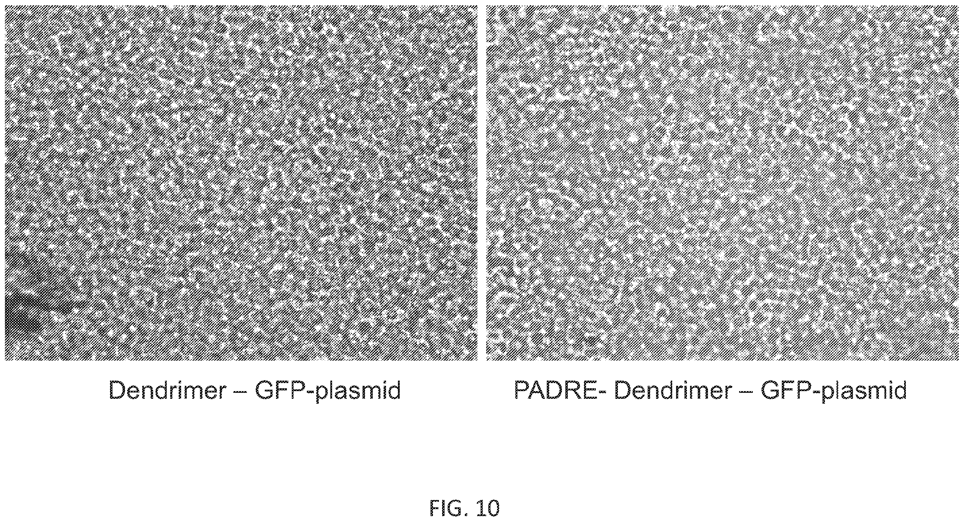

FIG. 10 is a pair of micrographs of Baboon PBMC transfected with dendrimer complexed with GFP-encoding plasmid (left panel) and cells transfected with PADRE-dendrimer complexed with GFP-encoding plasmid (right panel). PBMC of Baboon transfected with dendrimer complexed with GFP-plasmid (left panel) and cells transfected with PADRE-dendrimer complexed with GFP-plasmid (right panel). The flurescent microscope images were taken one day post addition of PDD/GFP-plasmid or control complex to Baboon PBMC. The image shows high efficacy of targeted delivery of the plasmid and the expression of the gene encoded by the plasmid via PDD.

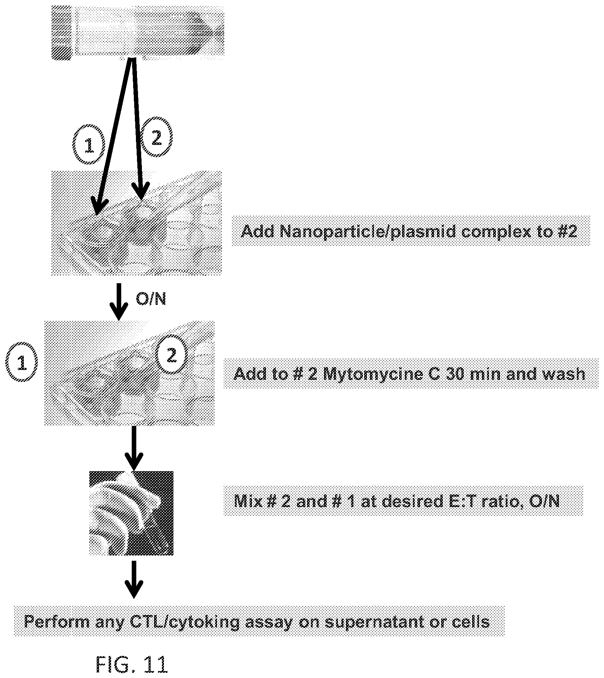

FIG. 11 is a schematic presentation of a protocol for *in vitro* transfection of human APCs among a population of PBMCs with a universal DR binding peptide-derivatized dendrimer as described herein. These transfections result in the processing and presentation of T cell epitopes in APCs, converting them into syngeneic APCs expressing the antigen of interest (referred to herein as "target cells").

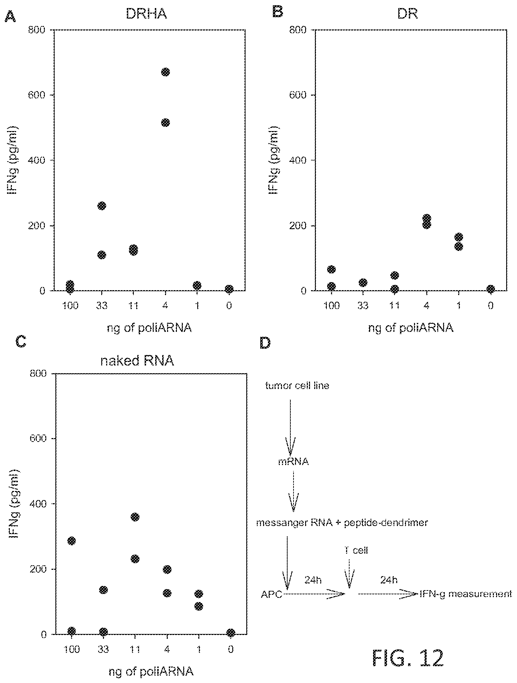

FIG. 12 is a series of graphs showing feasibility of using messenger RNA as a source of antigen for immunomonitoring of the immune response. Briefly, messenger RNA (mRNA) was isolated from the Balb/c murine mammary tumor 4T1 expressing the Haemoagglutinin antigen as artificial tumor associated antigen. mRNA was loaded on HA conjugated dendrimer or, as a control, on unconjugated dendrimer. The mRNA-HA conjugated dendrimers (A), the mRNA conjugated dendrimers (B) or the mRNA alone (C) were used to transfect splenocytes at different concentrations. 24 hours later, cells were washed and incubated with syngeneic CD8⁺ T cells against the MHC class I restricted Hemagglutinin epitope. IFN-gamma ELISA (Enzyme Linked ImmunoSorbent Assay) was used as a read out of CTL activity. The data clearly show that even using an extremely low quantity of RNA (4 ng), the use of HA-conjugated dendrimer (A), allowd the functional detection of CTL activity while only modest results are obtained with the controls (B and C). FIG. 12D is a schematic diagram of the experiment to evaluate the immune response.

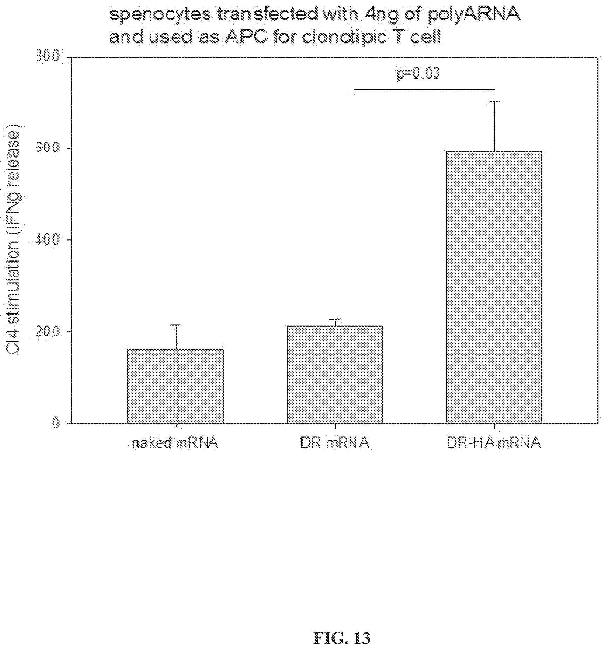

FIG. 13 is a graph showing results from an experiment in which splenocytes were transfected with 4 ng of polyA RNA and used as APCs for clonotypic T cells.

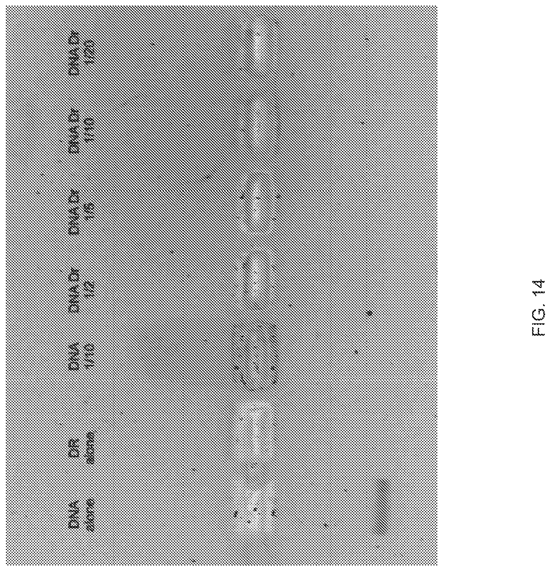

FIG. 14 is a photograph of an electrophoretic gel showing A) UV spectra of dendrimer, PADRE and dendrimer-PADRE. UV spectra of peptide-dendrimer was performed by standard methods. The phenylalanine peak seen for G5 dendrimer-PEDRE shows that the peptide, PADRE, is added to the dendrimer. B) Agarose gel electrophoresis and electrophoretic mobility analysis of dendrimer/DNA complex. Analysis of the complex formation of and the binding of PDD to DNA was performed by examining the retardation in the migration of the plasmid DNA during agarose gel electrophoresis. Peptide-derivatized-dendrimer (PDD)/ plasmid complexes were tested for their retainment of DNA in gel electrophoresis. Gel electrophoresis was performed for PDD/plasmid and controls: DNA alone, dendrimer alone, and PDD/plasmid samples at various ratios, 1:1, 1:2, 1:5, 1:10, 1:20 of (P:N). The PDD was able to retain DNA plasmid in ratios > (1:2).

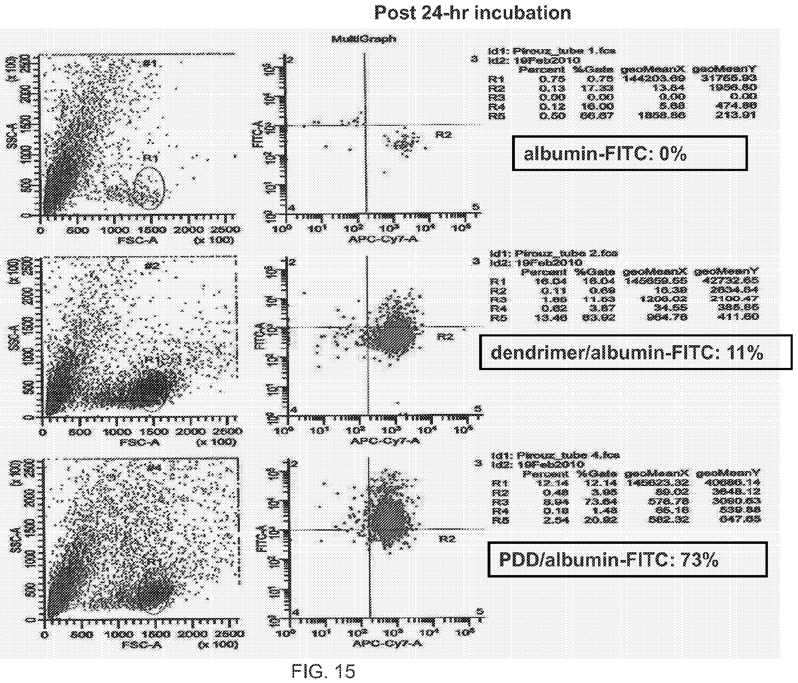

FIG. 15 is a series of flow cytometry Dot Plot diagrams showing cell-specific delivery of proteins/antigens. PDD/albumin-FITC was delivered into purified human B cells. PBMC were co-cultured with either of albumin-FITC alone, dendrimer/albumin-FITC, or dendrimer-PADRE (PDD)/albumin-FITC. Twenty four hours post-incubation in a 37°C/CO₂ incubator, cells with each treatment were analyzed by flow cytometry and gated for human B cells using anti-CD 19-APC. A ratio of 1:10 (w:w) of albumin-FITC and PDD or dendrimer was used. The high levels of delivery (73%) of PDD/albumin-FITC in human B cells clearly shows that the platform may be used with proteins/polypeptides or their like antigens.

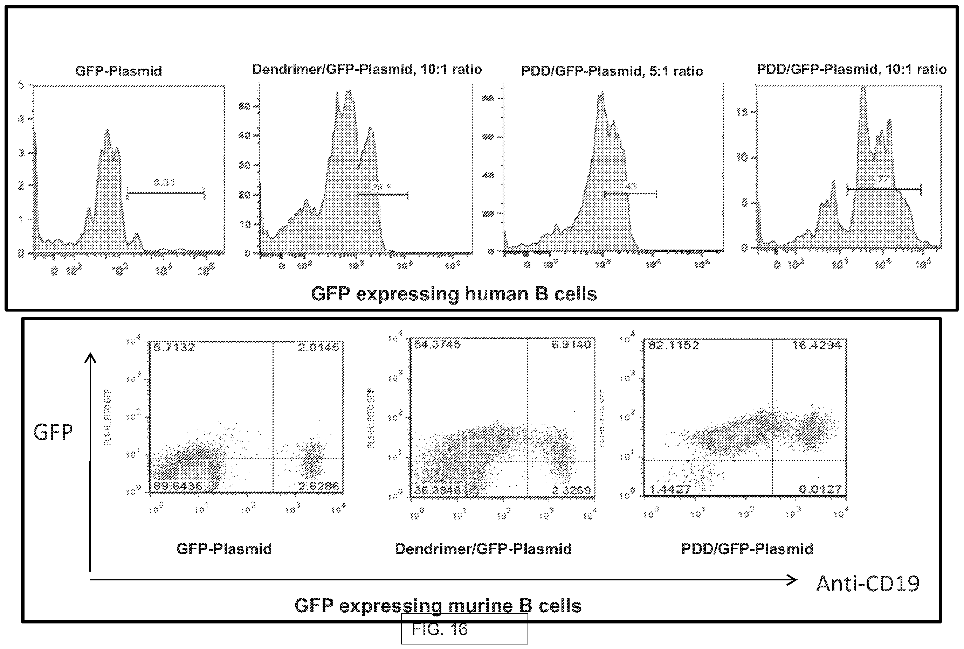

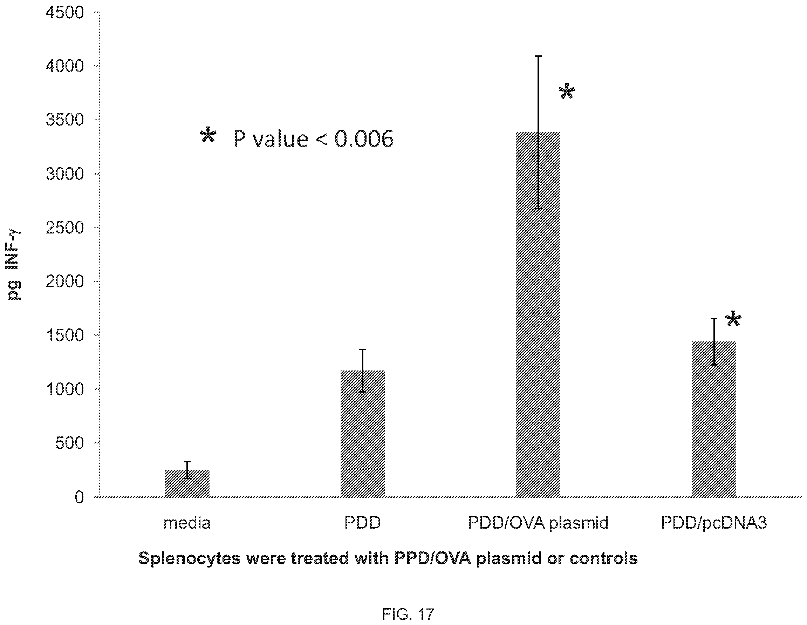

FIG. 16 is a series of flow cytometry histograms showing *in vitro* transfection of human B cells (CD19), upper panel, and mice splenocytes population, lower panel, with PDD PADRE-dendrimer(PDD)/GFP-Plasmid. In the upper panel, purified human B cells were co-cultured with either of GFP plasmid alone, dendrimer/GFP plasmid] or dendrimer-PADRE (PDD)/GFP plasmid at indicated P:N ratios. Twenty four hours post-incubation in a 37°C / CO₂ incubator, cells with each treatment were analyzed by flow cytometry for the expression of GFP protein. The high levels of delivery (77%) of PDD/GFP plasmid in human B cells clearly shows that the platform efficiently delivers plasmids into B cells and results in the expression of encoded protein/antigen. The lower panel shows a flow cytometry Dot Plot diagram when similar experiments were performed with splenocytes of C57BL naive mice and similarly shows the GFP transfection of CD-19 positive cells (B cells). FIG. 17 is a graph showing generation of APCs expressing antigen. Six to eight week old Female C57BL mice, in groups of five, were immunized twice with OVA protein in TiterMax (Sigma). Ten days post last immunization, the splenocytes of immunized mice were collected and plated at 1 million cells per well in four wells of a 24-well plate in RPMI with 10% FBS, the wells were labeled as "media alone", "PADRE-dendrimer (PDD) alone", "PADRE-dendrimer(PDD)/control-plasmid", and "PADRE-dendrimer(PDD)/OVA-plasmid". Five microgram of plasmids complexed with PADRE-dendrimer (in 1:10 ratio) was added to appropriate wells (target cells). The morning after, each treated/transfected cells were added to untreated splenocytes of same mouse in separate wells. Twenty four hours after stimulation, the levels of INF-γ were detected using ELISA (Thermo) in the supernatants. The levels of IFN-were significantly (P value < 0.006) higher in wells that contained splenocytes treated with [PDD/OVA-plasmid] than all controls which shows the kit may be used for evaluation of T cell responses upon vaccination. The induction of T cell responses were verified by challenge experiments using 50,000 B16-OVA as well as by OVA peptide stimulation (not shown). FIG. 17 shows that vaccination efficacy was measured in mice; note the significant differences in the levels of IFN-γ in vaccinated mice.

### DETAILED DESCRIPTION

Described herein are targeted nanoparticle-based methods, assays, kits and compositions for transfection of APCs with an antigen or a nucleic acid encoding an antigen in a sample of human PBMCs (or a human sample containing PBMCs) that results in the processing and presentation of a broad repertoire of antigen epitopes on the surface of the APCs. These APCs (referred to herein as "target cells") provide properly configured (i.e., native) pathogen epitopes with universal applicability for accurate monitoring of cellular immune responses to any vaccine or other therapy or intervention. The nanoparticles are complexed to an antigen or a nucleic acid encoding an antigen, and a universal DR binding peptide (e.g., a T helper epitope)-derivatized dendrimer which specifically binds to MHC class II molecules expressed on APCs to deliver specific epitopes of the antigen against which a subject is vaccinated, for example. The targeted nanoparticle-based methods, assays, kits and compositions can be also used for examining the immunotoxicity of a biologic or drug in a subject or population of subjects. The assays, kits, compositions and methods described herein provide a low-cost approach for rapid generation of reagents and accurate profiling of immunological responses to infections, immunizations or other therapies or interventions, as well as a low-cost and efficient approach for examining the immunotoxicity of a biologic or drug in a subject or population of subjects.

In a typical composition, a charged (e.g., positively-charged), highly branched polymeric dendrimer is conjugated to an MHC targeting, universal, Pan DR binding peptide or a combination of such peptides (e.g., an epitope such as the PADRE or Influenza HA T helper epitope: SFERFEIFPKEC (SEQ ID NO:28), etc.) and conjugated or bound to a particular antigen or allergen or a nucleic acid (e.g., DNA, RNA) encoding the antigen against which a subject is to be or has been vaccinated. The antigen may be a protein or peptide of any pathogenic origin, e.g., bacterial, fungal, protozoan, or viral origin, or a fragment derived from these antigens, a carbohydrate, or a carbohydrate mimetic peptide. A charged (e.g., positively-charged), highly branched polymeric dendrimer can be conjugated to two or more different antigens or allergens and similarly, can be conjugated to two or more nucleic acids that each encode a different antigen. The dendrimer makes a complex (conjugation) with antigens (nucleic acids or proteins) based on the opposite charge of the dendrimer (positive) and that of antigen (negative) or the conjugation may be a covalent chemical linkage.

In one embodiment, a nanoparticle-based method to deliver antigens to APCs for assessing or monitoring an immune response in a subject (e.g., an immune response against a vaccine, against a biologic or drug, etc.) as described herein includes conjugating a nucleic acid (e.g., a DNA or RNA sequence) encoding an allergen, antigen or an antigenic peptide or polypeptide to a charged (e.g., positively-charged), highly branched polymeric dendrimer (e.g., PADRE-derivatized dendrimer (PDD)) that is also conjugated to at least one universal DR binding/MHC targeting peptide such as the ones listed below (e.g., in the Table 1). Negatively-charged plasmids bind naturally to the positively-charged universal DR binding peptide-dendrimers (e.g., PADRE-dendrimers), while allergen or peptide or polypeptide antigens can be chemically linked to the universal DR binding peptide-dendrimers if they are not negatively-charged. In other embodiments, a dendrimer is negatively-charged for binding to positively-charged proteins and peptides. Surface-exposed allergen(s), antigen(s) or nucleic acid(s) encoding an antigen(s) may be conjugated to the dendrimers by any suitable means known in the art. Conjugation methods include chemical complexation, which may be either ionic or nonionic in nature, electrostatic binding, or covalent binding.

In a typical method of detecting an immune response against a therapy or intervention in a subject, samples are obtained from the subject prior to and after receiving the therapy or intervention. For example, if the therapy or intervention is vaccination, a sample is obtained from the subject prior to vaccination, and a sample is obtained from the subject after the subject has been vaccinated. In a method of detecting an immune response against a vaccine that includes at least one antigen in a subject, the method includes the following several steps. A preimmune PBMC preparation or sample is obtained from the subject before the subject has been vaccinated (referred to herein as "a first sample"). The preimmune PBMC is obtained via standard methods and cryopreserved in DMSO at concentrations of approxuimately 5 million cells per vial. PBMC may be stored in liquid nitrogen or -80C freezer depending on the duration of vaccination. On the day of the experiment, the preimmune PBMC is thawed and cultured under standard conditions (e.g., quick thaw at 37°C followed by one wash step to remove DMSO by spinning cells at 400g for 5 min). The preimmune cells are then treated with PDD/antigen as explained in forthcoming sections. This first sample is divided into at least two portions, referred to herein as a first portion of the first sample (also referred to as "target cells"), and a second portion of the first sample (also referred to as "effector cells"). Each portion typically includes approximately 5 million PBMCs (e.g., 1 million, 2 million, 3 million, 4 million, 5 million, 6 million PBCs, etc.). The first portion of the first sample is treated with (contacted with, mixed with) highly branched polymeric dendrimers conjugated to an MHC targeting and universal DR binding peptide as described herein. The dendrimers are also conjugated to the at least one antigen or a nucleic acid encoding the at least one antigen. The dendrimers conjugated to an MHC targeting and universal DR binding peptide and at least one antigen or a nucleic acid encoding the at least one antigen are prepared as described herein. After being added to the first portion of the first sample, the dendrimers enter APCs, and the APCs process and present the antigen on their surfaces in combination with MHC class II molecules. The resultant APCs are referred to herein as "target cells." PBMCs may be frozen in DMSO using standard methods of freezing PBMCs, and these PBMCs can be used as a reference (background reference of default levels of immune responses to compare with) of T cell responses before vaccination, immunotherapy or other interventions. Effector cells and target cells can be viably frozen in multiple aliquots for future use.

The first and second portions of the first sample are incubated in a 37°C/5% CO₂ incubator overnight. In some embodiments, mitomycin-C may be added to the target cells because mitomycin C treatment eliminates the proliferation of APCs, and reduces cytokine expression (background). In such embodiments, after 24 hours from the beginning of the incubation, depending on the type of nucleic acid conjugated to the dendrimers (this time period is generally optimized for different types of nucleic acids, e.g., different plasmids), mitomycin C is added to the first portion of the first sample (to the target cells) for approximately 30 minutes. Then, the first and second portions of the first sample (the target cells and the effector cells) are washed, e.g., with fresh media, 10 minutes at 400 g. The first portion of the first sample and the second portion of the first sample are then mixed at one or more ratios (in one or more different containers, wells, tubes, plates, etc.) resulting in a first plurality of mixtures (the ratio or ratios are generally optimized for different types of nucleic acids, e.g., different plasmids). After a suitable incubation period (typically 6-48 hours, depending on the type of nucleic acid and other conditions), the mixtures are examined for the presence of and levels of one or more molecules or markers that is indicative of an immune response to the therapy or intervention. This is typically done by examining the supernatants of the first plurality of mixtures, e.g., detecting and measuring the level of the molecule or marker in the supernatants. For example, the mixtures can be examined for the presence of one or more cytokines, and the level of the one or more cytokines (e.g., IFN-γ) in each mixture can be determined (measured). Any suitable assay that measures T helper cell or B cell activation and proliferation and/or levels and expression of one or more molecules or markers (e.g., cytokines) that is indicative of an immune response to the vaccine or other therapy or intervention can be used. Examples of such assays include CTL and cytokine assays.

After the subject has received the vaccination, PBMCs are obtained from the subject, referred to herein as a "second sample." Depending on the type of response (effector or memory), a second sample is drawn from the individual, typically 7-30 days post-vaccination or intervention to measure T cell immune responses, however, the T cell responses may be measured on any later time for their durability. This second sample is divided into at least two portions, referred to herein as a first portion of the second sample, and a second portion of the second sample. Each portion typically includes approximately 5 millions PBMCs (e.g., 1 million, 2 million, 3 million, 4 million, 5 million, 6 million PBCs, etc.). The first portion of the second sample is treated with (contacted with, mixed with) the dendrimers described above (i.e., highly branched polymeric dendrimers conjugated to an MHC targeting and universal DR binding and at least one antigen or a nucleic acid encoding the at least one antigen). After being added to the first portion of the second sample, the dendrimers enter APCs, and the APCs process and present the antigen on their surfaces in combination with MHC class II molecules. The resultant APCs are referred to herein as "target cells." The first and second portions of the second sample are incubated in a 37°C/5% CO₂ incubator overnight. After 24 hours from the beginning of the incubation, depending on the type of nucleic acid conjugated to the dendrimers (this time period is generally optimized for different types of nucleic acids, e.g., different plasmids), mitomycin C is added to the first portion of the second sample (to the target cells) for approximately 30 minutes. Then, the first and second portions of the second sample (the target cells and the effector cells, respectively) are washed, e.g., with fresh media, 10 minutes at 400 g. The first portion of the second sample and the second portion of the second sample are then mixed at one or more ratios (in one or more different containers, wells, tubes, plates, etc.) resulting in a second plurality of mixtures (the ratio or ratios are generally optimized for different types of nucleic acids, e.g., different plasmids) under conditions that allow for stimulation of existing specific T cells in the PBMCs of the individual which results in the production of cytokines as well as proliferation of T cells that are specific for the antigen if the vaccination or other intervention or therapy was effective in promoting an immune response to the antigen. After a suitable incubation period (typically 6-48 hours, depending on the type of nucleic acid and other conditions), the mixtures are examined for the presence and level(s) of one or more molecules or markers that is indicative of an immune response to the therapy or intervention. This is typically done by examining the supernatants of the second plurality of mixtures, e.g., detecting and measuring the level of the molecule or marker in the supernatants. For example, the mixtures can be examined for the presence of one or more cytokines, and the level of the one or more cytokines (e.g., IFN-γ) in each mixture can be determined (measured). As mentioned above, any suitable assay that measures T helper cell or B cell activation and proliferation and/or levels and expression of one or more molecules or markers (e.g., cytokines) that is indicative of an immune response to the vaccine or other therapy or intervention can be used. Examples of such assays include CTL and cytokine assays.

In this method, the level of the at least one molecule or marker that is indicative of an immune response in the first plurality of mixtures is compared to the level of the at least one molecule or marker in the second plurality of mixtures. If the vaccine was effective in promoting an immune response to the at least one antigen, the second portion of the second sample will include reacting, primed, sensitized T cells specific for the antigen. Thus, a comparison is made between the levels of the one or more molecules or markers (e.g., IFN-γ) in the supernatants of the first plurality of mixtures, and the supernatants of the second plurality of mixtures, and higher levels of the at least one molecule or marker in the second plurality of mixtures than in the first plurality of mixtures is correlated with an immune response to the vaccine in the subject. For example, if the vaccine was effective in promoting an immune response to the at least one antigen, the levels of IFN-γ will be higher in the supernatants of the second plurality of mixtures then the levels of IFN-γ in the first plurality of mixtures.

Levels of the cytokines and/or extent of T cell proliferation/activation proportionally correlate with the T cell responses in the PBMCs from the subject. Thus, by measuring the levels of cytokines and extent of T cell proliferation/activation, whether or not a vaccine was effective in mounting an immune response against the antigen, and the extent of the immune response mounted, can be determined. For example, if a subject is vaccinated with antigen X, the nanoparticles conjugated to the antigen against which the vaccine was raised (antigen X), or a nucleic acid encoding the antigen (antigen X) incubated with PBMCs of the subject cause the subject's APCs to process and present a natural T cell epitope repertoire of antigen X, a process that converts PBMCs to "target cells" expressing antigen X. Upon co-culturing of the effector cells with the target cells, if the effector cells include any reacting T cells specific for the antigen, the T cells will proliferate and produce related cytokines. Levels of cytokines (such as IFN-γ) are assessed by any suitable method, including ELISA, ELISPOT, or intracellular cytokine assay. The extent of T cell proliferation is quantified by any proliferation assay such as i) the 3H-Thymidine assay which is based on radioactive thymidine incorporation, or by ii) 5,6-carboxyfluorescein diacetate succinimidyl ester (CFSE)-based T Cell Proliferation Assays in which cells are labeled with the fluorescent dye (CFSE). In the latter assay, those cells that proliferate in response to the antigen show a reduction in CFSE fluorescence intensity, and the percentage of proliferating CD4+ T cells may be determined using flow cytometry, for example.

In a method of assessing the immunogenicity of a drug or biologic, nanoparticles are prepared as described above, with the variation that they are complexed with or conjugated to the drug, or a portion thereof, or the biologic, or a portion thereof. The resultant nanoparticles are contacted with at least one subject's PBMCs (e.g, PBMCs from 1, 2, 3, 4, 5, 6, 7, 8, 9, 10, 100, 1000, 10,000, etc. subjects) and assayed as described above ("target cells" and "effector cells" are from the same individual). If elevated cytokine levels and/or elevated levels or activation of T cells or B cells or proliferation specific for the drug or biologic are detected in an assay as described herein, the drug or biologic is determined to be immunotoxic, because the drug or biologic has caused a T cell or B cell response in the subject(s). This embodiment is particularly useful for assisting medical practitioners in determining whether or not to administer the biologic or drug to a subject or a plurality of subjects, as well as for assisting the manufacturer of the biologic or drug in determining the efficacy of the biologic or drug in a subject or a population of subjects.

A dendrimer conjugated to a universal DR binding peptide (e.g., T helper epitope) as described herein can be multivalent; it can present (be complexed with) more than one copy or type of antigen or nucleic acid or drug or biologic or allergen on its surface. The one or more copies or types of antigens or nucleic acids or drugs or biologics or allergens can be attached to the dendrimer via two or more separate linkers or spacers, or via a common linker or spacer. A nanoparticle for assessing the efficacy of a vaccine or other therapy or intervention as described herein can be used to assess the efficacy of any vaccine or therapy or intervention. Similarly, a nanoparticle for assessing the efficacy of (e.g., the immunotoxicity of) a biologic or drug as described herein can be used to assess the efficacy of (e.g., the immunotoxicity of) any biologic or drug.

The below described preferred embodiments illustrate adaptations of these compositions, assays, kits and methods. Nonetheless, from the description of these embodiments, other aspects of the invention can be made and/or practiced based on the description provided below.

### Biological Methods

Methods involving conventional molecular biology techniques are described herein. Such techniques are generally known in the art and are described in detail in methodology treatises such as Molecular Cloning: A Laboratory Manual, 3rd ed., vol. 1-3, ed. Sambrook et al., Cold Spring Harbor Laboratory Press, Cold Spring Harbor, N.Y., 2001; and Current Protocols in Molecular Biology, ed. Ausubel et al., Greene Publishing and Wiley-Interscience, New York, 1992 (with periodic updates). Immunology techniques are generally known in the art and are described in detail in methodology treatises such as Advances in Immunology, volume 93, ed. Frederick W. Alt, Academic Press, Burlington, MA, 2007; Making and Using Antibodies: A Practical Handbook, eds. Gary C. Howard and Matthew R. Kaser, CRC Press, Boca Raton, Fl, 2006; Medical Immunology, 6th ed., edited by Gabriel Virella, Informa Healthcare Press, London, England, 2007; and Harlow and Lane ANTIBODIES: A Laboratory Manual, Cold Spring Harbor Laboratory Press, Cold Spring Harbor, NY, 1988. Conventional methods of gene transfer and gene therapy may also be adapted for use in the present invention. See, e.g., Gene Therapy: Principles and Applications, ed. T. Blackenstein, Springer Verlag, 1999; and Gene Therapy Protocols (Methods in Molecular Medicine), ed. P.D. Robbins, Humana Press, 1997. Construction and use of vaccines as well as PAMAM dendrimers is also described, for example, in Arashkia et al., Virus Genes 40 (1): 44-52, 2010; Velders et al., J Immunol. 166:5366-5373, 2001; and S. Chauhan, N. K. Jain, P. V. Diwan. (2009) Pre-clinical and behavioural toxicity profile of PAMAM dendrimers in mice. Proceedings of the Royal Society A: Mathematical, Physical and Engineering Sciences (Online publication date: December 3, 2009).

### Synthesis of Dendrimers Conjugated to Nucleic Acids, Peptides, Polypeptides, Drugs or Biologics

Charged, circular tree-like structures such as dendrimers act as scaffolds to condense DNA, and a fully positively-charged dendrimer is preferable for developing strong electrostatic interactions with a negatively-charged DNA or RNA. A resulting dendrimer/universal DR binding peptide/DNA complex, for example, has a net charge depending on the adjustable N/P ratio (amine to phosphate or charge ratio). Described herein are dendrimers having conjugated thereto at least one universal DR binding peptide (e.g., an epitope such as the PADRE or Influenza HA T helper epitope: SFERFEIFPKEC (SEQ ID NO:28)) and an allergen, an antigen, a nucleic acid encoding an antigen, a drug, a biologic, or a portion thereof. The at least one universal DR binding peptide is conjugated to the exterior surface of the dendrimer such that the at least one universal DR binding peptide specifically binds to PAPCs. In one embodiment, dendrimers are conjugated to at least one of either or a combination (e.g., 1, 2, 3, 4, 5, etc.) of these peptides: SSVFNVVNSSIGLIM (SEQ ID NO:29), FNNFTVSFWLRVPKVSASHLE (SEQ ID NO:30), QYIKANSKFIGITEL (SEQ ID NO:31), KLLSLIKGVIVHRLEGVE (SEQ ID NO:32), LSEIKGVIVHRLEGV (SEQ ID NO:33), DGVNYATGNLPGCSA (SEQ ID NO:34), ENDIEKKICKMEKCSSVFNV (SEQ ID NO:35), NLGKVIDTLTCGFA (SEQ ID NO:36), GQIGNDPNRDIL (SEQ ID NO:37), IDVVDSYIIKPIPALPVTPD (SEQ ID NO:38), ALNNRFQIKGVELKS (SEQ ID NO:39), AKXVAAWTLKAAA (SEQ ID NO:2), PRYISLIPVNVVAD (SEQ ID NO:40), and/or VATRAGLVMEAGGSKVT (SEQ ID NO:41) and a peptide or polypeptide antigen, or allergen. In this embodiment, a dendrimer is typically conjugated to or bound to (e.g., via an electrostatic binding) a plurality of the peptide or polypeptide antigen or allergen.

Plasmids endoding antigens, subunits, vaccines and protein/glycoprotein antigens readily make a complex with PDD due to their negative net charge or pockets of negative charge present in their structure. However, when the antigen or compound does not contain a negative "net charge" or "negatively charged pockets in their structure," they may be covalently conjugated to the PDD. Examples are small-size preservatives, compounds to increase stability of drugs and/or biologics or to increase tissue penetration of drugs/biologics, compounds to induce depot effects of drugs or biologics, compounds to change charge or the solubility of drugs or biologics, or any compound or antigen that does not readily make complex with PDD. Multiple antigens may be complexed with the dendrimer/universal DR binding peptide/nucleic acid complexes described herein to enhance the immunomonitory capacity of the platform via assessing T cell responses against different antigens or components of a drug. In another embodiment, dendrimers are conjugated to universal DR binding peptides (e.g., PADRE peptides) and bound to a nucleic acid encoding an antigen. In this embodiment, the dendrimers can be prepared and conjugated to a universal DR binding peptide (e.g., an epitope such as the PADRE or Influenza HA T helper epitope: SFERFEIFPKEC (SEQ ID NO:28) and bound to the nucleic acid (e.g., DNA, RNA) using any suitable method. In a further embodiment, via the dendrimer component, the composition is complexed with or conjugated to a drug or biologic or a portion thereof. In this embodiment, a dendrimer moiety (the dendrimer component that makes electrostatic bonds with the antigen or DNA or RNA) of the platform is typically conjugated to or bound to (e.g., via an electrostatic binding) a plurality of the drug or biologic. Such complexes composed of the drug(s) or biologic(s) and the "peptide -derivatized-dendrimer, or PDD", where the peptide is a universal DR binding peptide (e.g., an epitope such as tetanus toxin 582-599, the PADRE peptide or Influenza HA T helper epitope: SFERFEIFPKEC (SEQ ID NO:28) or a combination of such peptides)) can be produced by any suitable method. An entire drug, protein, biologic, or nucleic acid encoding for the entire protein or biologic may be bound (conjugated) to the PDD. Alternatively, a portion or subunit of the protein, drug or biologic, or a truncated form of a nucleic acid encoding the protein or biologic may be bound (conjugated) to the PDD. In a typical embodiment of determining the immunotoxicity of a drug or biologic, such complexes are added to PBMCs of an individual(s) to assess/predict whether or not the individual will mount a T cell immune response against the drug or biologic prior to administration of the drug or biologic to the individual(s) (e.g., by oral administration, injection, or any other forms of administering drugs or biologics).

Methods of producing and using dendrimers are well known in the art and are described, for example, in Zhang J-T et. al. Macromol. Biosci. 2004, 4, 575-578, and U.S. Patent Nos. 4,216,171 and 5,795,582. See also: D.A. Tomalia, A.M. Naylor, and W.A. Goddard III, "Starburst Dendrimers: Molecular-Level Control of Size, Shape, Surface Chemistry, Topology, and Flexibility from Atoms to Macroscopic Matter", Angew. Chem. Int. Ed. Engl. 29 (1990), 138-175. In the experiments described herein, PAMAM dendrimers were used. However, any suitable charged (e.g., positively-charged), highly branched polymeric dendrimer can be used. Examples of additional positively charged, highly branched polymeric dendrimers include poly(propylene imine) (PPI) dendrimers or, more generally, any other dendrimers with primary amine groups on their surfaces.

The PADRE-dendrimers (PADRE-derivatized dendrimers) described herein can be prepared by any suitable method. Methods of making and using PADRE are known in the art. See, for example, U.S. Patent No. 5,736,142. To produce the PADRE peptides described in U.S. Patent No, 5,736,142, a strategy initially described by Jardetzky et al. (EMBO J. 9:1797-1083, 1990) was used, in which anchor residues that contain side chains critical for the binding to MHC are inserted into a poly-alanine peptide of 13 residues. PADRE peptides can be prepared according to the methods described in U.S. Patent No. 5,736,142, for example, or they can be purchased (e.g., from Anaspec, Inc., Fremont, CA). Because of their relatively short size, the PADRE peptides (or other universal DR binding peptide) can be synthesized in solution or on a solid support in accordance with conventional techniques. Various automatic synthesizers are commercially available and can be used in accordance with known protocols. Alternatively, recombinant DNA technology may be employed wherein a nucleotide sequence which encodes a universal DR binding peptide (e.g., T helper epitope) is inserted into an expression vector, transformed or transfected into an appropriate host cell and cultivated under conditions suitable for expression. These procedures are generally known in the art, as described generally in Sambrook et al., *(supra).* PADRE peptides as described herein may include modifications to the N- and C-terminal residues. As will be well understood by the artisan, the N- and C-termini may be modified to alter physical or chemical properties of the peptide, such as, for example, to affect binding, stability, bioavailability, ease of linking, and the like. The universal DR binding peptides (e.g., PADRE peptides) described herein may be modified in any number of ways to provide desired attributes, e.g., improved pharmacological characteristics, while retaining substantially all of the biological activity of the unmodified peptide.

In the experiments described herein, the PADRE-dendrimer conjugate was made by simple amide coupling between the -COOH terminus of the PADRE peptide and one of the dendrimer amine groups. The PADRE peptide (Ac-D-Ala-Lys-Cha-Val-Ala-Ala-Trp-Thr-Leu-Lys-Ala-Ala-Ala-D-Ala-Ahx-Cys-OH) (SEQ ID NO:1, Ac= acetylated; D-Ala = D-alanine; Cha = cyclohexylalanine; Ahx = aminohexanoic acid) was purchased from Anaspec, Inc., (Fremont, CA) in its acetylated form in order to protect the amine terminus and prevent its reaction. The purchased peptide had a minimum purity of 95%. The amide coupling reaction was carried out under standard conditions (see FIG. 1, bottom schematic) in DMF solution. In order to control the number of PADRE epitopes attached to the surface of each dendrimer, a 2:1 peptide/dendrimer challenge ratio was used in the reaction, seeking attachment of just a few peptides per dendrimer in order to keep most of the amine groups free to develop large positive charges on the dendrimer. In a typical embodiment, a plurality of PADRE-dendrimer conjugates as described herein will be a distribution of dendrimers containing 0, 1, 2, 3, etc., PADREs (or other peptide) attached thereto. Relative populations are expected to follow the Poisson distribution. The PADRE, aKXVAAWTLKAAa (SEQ ID NO:2) binds with high or intermediate affinity (IC₅₀<1,000 nM) to 15 out of 16 of the most prevalent HLA-DR molecules ((Kawashima et al., Human Immunology 59:1-14 (1998); Alexander et al., Immunity 1:751-761 (1994)). However, other peptides which also can bind MHC class II and activate CD4 T helper cells in most humans may also be used to tag the dendrimer.

Examples of peptides include but are not limited to: peptides from tetanus toxoid (TT) (e.g., peptide 830-843); the "universal" epitope described in Panina-Bordignon et al., (Eur. J. Immunology 19:2237-2242 (1989)); and the following peptides that react with MHC class II of most human HLA, and many of mice: aKFVAAWTLKAAa (SEQ ID NO:3), aKYVAAWTLKAAa (SEQ ID NO:4), aKFVAAYTLKAAa (SEQ ID NO:5), aKXVAAYTLKAAa (SEQ ID NO:6), aKYVAAYTLKAAa (SEQ ID NO:7), aKFVAAHTLKAAa (SEQ ID NO:8), aKXVAAHTLKAAa (SEQ ID NO:9), aKYVAAHTLKAAa (SEQ ID NO: 10), aKFVAANTLKAAa (SEQ ID NO: 11), aKXVAANTLKAAa (SEQ ID NO:12), aKYVAANTLKAAa (SEQ ID NO:13), AKXVAAWTLKAAA (SEQ ID NO:2), AKFVAAWTLKAAA (SEQ ID NO:14), AKYVAAWTLKAAA (SEQ ID NO:15), AKFVAAYTLKAAA (SEQ ID NO:16), AKXVAAYTLKAAA(SEQ ID NO:17), AKYVAAYTLKAAA (SEQ ID NO:18), AKFVAAHTLKAAA (SEQ ID NO:19), AKXVAAHTLKAAA (SEQ ID NO:20), AKYVAAHTLKAAA (SEQ ID NO:21), AKFVAANTLKAAA (SEQ ID NO:22), AKXVAANTLKAAA (SEQ ID NO:23), and AKYVAANTLKAAA (SEQ ID NO:24) (a = D-alanine, X = cyclohexylalanine). Another example of an epitope that may be used is the HA peptide sequence SFERFEIFPKE (SEQ ID NO:25) (from the provirus PR8 virus HA) that binds to mouse Balb/c MHC classII IaD. Further examples of universal DR binding peptides include supermotifs such as Class II-associated invariant chain peptides (CLIPs), in particular CLIP p88-99 (SKMRMATPLLMQ (SEQ ID NO:42)), that bind to multiple HLA haplotypes.

Additional examples of universal DR binding peptides (e.g., T helper epitopes) are in Table 1. Such epitopes bind the majority of human HLA, for example, VATRAGLVMEAGGSKVT (SEQ ID NO:41), a T cell epitope from Mce2 Mycobacteriumtuberculosis), binds 9 different HLA DR: DRB1^{∗}0101 (DR1), DRB1^{∗}1501 (DR2), DRB1^{∗}0301 (DR3), DRB1^{∗}0401 (DR4), DRB1^{∗}1101 (DR5), DRB1^{∗}0701 (DR7), DRB1^{∗}0801 (DR8), or a Pan D binding peptide. PADRE is capable of binding to 6 selected DRB1 subtypes (Alexander J, Immunity vol. 1:751-761, 1994), and to APCs of PBMCs (Neumann, Journal of Cancer vol. 112:661-668, 2004) and was shown to bind to both human and murine MHC class II (Kim, J. Immunol. Vol. 180:7019-7027, 2008). As described herein, a typical universal DR binding peptide is PADRE peptide (e.g., aKXVAAWTLKAAaZC (SEQ ID NO:43), with X = L-cylohexylamine, Z = aminocaproic acid, and the remaining one-letter symbols representing the usual amino acids). As mentioned above, one or a combination of all of the following peptides can be used: SSVFNVVNSSIGLIM (SEQ ID NO:29), FNNFTVSFWLRVPKVSASHLE (SEQ ID NO:30), QYIKANSKFIGITEL (SEQ ID NO:31), KLLSLIKGVIVHRLEGVE (SEQ ID NO:32), LSEIKGVIVHRLEGV (SEQ ID NO:33), DGVNYATGNLPGCSA (SEQ ID NO:34), ENDIEKKICKMEKCSSVFNV (SEQ ID NO:35), NLGKVIDTLTCGFA (SEQ ID NO:36), GQIGNDPNRDIL (SEQ ID NO:37), IDVVDSYIIKPIPALPVTPD (SEQ ID NO:38), ALNNRFQIKGVELKS (SEQ ID NO:39), AKXVAAWTLKAAA (SEQ ID NO:2), PRYISLIPVNVVAD (SEQ ID NO:40), and/or VATRAGLVMEAGGSKVT (SEQ ID NO:41).

**Table 1. Examples of Universal DR Binding**

| Peptides | |
|---|---|
| Description | Amino acid sequence |
| measles 289-302 | LSEIKGVIVHRLEGV (SEQ ID NO:33) |
| tetanus toxin 582-599 | VDDALINSTKIYSYFPSV (SEQ ID NO:44) |
| tetanus toxin 830-844 | QYIKANSKFIGITEL (SEQ ID NO:31) |
| Anaplasma marginale | SSAGGQQQESS (SEQ ID NO:45) |
| circumsporozoite (CS) protein | ENDIEKKICKMEKCSSVFNV (SEQ ID NO:35) |
| influenza HA B epitope | SKAFSNCYPYDVPDYASL (SEQ ID NO:46) |
| Pfg27 (Plasmodium falciparim, sexual stage) | IDVVDSYIIKPIPALPVTPD (SEQ ID NO:38) |
| PvMSP-1 (Plasmodium vivax merozoit) | LEYYLREKAKMAGTLIIPES (SEQ ID NO:47) |
| Mce2 *(Mycobacteriumtuberculosis)* | PRYISLIPVNVVAD (SEQ ID NO:40) |
| Mce2 *(Mycobacteriumtuberculosis)* | VATRAGLVMEAGGSKVT (SEQ ID NO:41) |
| PADRE | AKXVAAWTLKAAA (SEQ ID NO:2) |

The product was purified by dialysis against pure water for at least 24 h and then dried under vacuum. The collected product, a clear oil, was characterized by ¹H NMR, UV-Vis and MALDI-TOF mass spectroscopy. The NMR spectra of the PADRE-dendrimer conjugate shows large peaks corresponding to the dendrimer protons and a small set of peaks for the peptide protons. The MALDI-TOF mass spectrum of the PADRE-dendrimer conjugate shows a peak at a m/z ratio ca. 3,000 units higher than the peak observed for the dendrimer on its own. The excess mass corresponds to approximately 2 peptide epitopes. The UV-Vis spectrum of the conjugate shows a clear absorption in the wavelength range where tryptophan absorbs.

Complexation of plasmid DNA with the PADRE-dendrimer conjugate was done by mixing the two components in aqueous solution buffered at physiological pH with PBS. The buffer or media to make the complex of PDD (peptide-derivatized-dendrimer) and plasmid or antigen contains physiological buffer, typically with a pH of 7.4. Examples are i) a water-based salt solution containing sodium chloride, sodium phosphate, and (in some formulations) potassium chloride and potassium phosphate such as PBS (phosphate Buffered Saline), or ii) a media that contains Eagle's Minimal Essential Medium, buffered with HEPES and sodium bicarbonate, and supplemented with hypoxanthine, thymidine, sodium pyruvate, L-glutamine, and less than 10% serum bovine albumin or individual serum proteins including insulin and/or teansferrin with 100 mg/L CaCl₂ where the endotoxin level is less than 1.0 EU/mL.

An example of a protocol for forming the complex of PDD (peptide-derivatized-dendrimer) and plasmid or antigen includes the following steps. First, plate PBMCs in 4 wells of a 24-well plate at 1 million per ml, 1 ml per well, number them as 1, 2, 3, and 4. Second, dilute 5 µg of plasmid(s), or antigen(s) in 800 µl of the above-described buffer. Third, dilute 35 µg of PDD in 200 µl of the buffer, add drop-wise to the solution of 5 µg of plasmid(s), or antigen(s) in 800 µl of the above-described buffer, while shaking the container, and incubate at room temperature for 10 minutes. Fourth, add this mixture to well number 4 (from step 1). Next, add 35 µg of the PDD to well number 3, and optionally to well number 2 if you wish to use irrelevant plasmid/antigen and well number 1 for the media alone.

Typical N/P (amine to phosphate) ratios are 10:1. Gel electrophoresis is used to show complete complexation of the DNA. At physiological pH values, the amino groups (-NH2) are protonated, affording a high positive charge to the dendrimers and making them particularly well-suited for the delivery of negatively-charged DNA or RNA into cells. In aqueous solution, the positively-charged dendrimers and the negatively-charged nucleic acids give rise to condensates or nanoparticles which can penetrate and traverse biological membranes with relative ease.

Dendrimers that are conjugated to T helper epitopes other than PADRE are typically prepared by a method similar to that described above for PADRE-derivatized dendrimers. For example, the acid terminus of the peptide can be covalently attached to one of the amine groups on the dendrimer surface by a number of well-known synthetic methods, such as amidation using carbodiimides as activating reagents. As another example, attachment of these peptides to amino-terminated dendrimers is performed using two synthetic routes. The amino terminus of the peptide epitope is protected by acetylation. The first route uses the carboxylic acid of the terminal cysteine residue to achieve attachment via standard amidation chemistry. The second route takes advantage of the cysteine's thiol (if present on the peptide, otherwise may be added) to react it with the alkene groups added to the dendrimer surface by previous treatment with maleimide. Both routes allow the functionalization of dendrimers with epitopes. Up to several peptide epitopes (e.g., 2, 3, 4, 5, 6, etc.) per dendrimer will enhance the targeting property of the DNA delivery agents, improving their properties for vaccination purposes. However, it is important to leave a large number of unreacted amine groups so that the dendrimer will acquire a large positive charge via protonation at physiological pH values. Dendrimers as described herein can be conjugated to any T helper epitope. An example of an additional universal DR binding peptide is Influenza HA.

Generally, generation-5 (G5) dendrimers are used in the compositions, kits, assays and methods described herein. However, other generation dendrimers (see Table 2) can be used.

**Table 2 PAMAM Dendrimers**

| Generation | Molecular Weight | Diameter (nm) | Surface Groups |
|---|---|---|---|
| 0 | 517 | 1.5 | 4 |
| 1 | 1,430 | 2.2 | 8 |
| 2 | 3,256 | 2.9 | 16 |
| 3 | 6,909 | 3.6 | 32 |
| 4 | 14,215 | 4.5 | 64 |
| 5 | 28,826 | 5.4 | 128 |
| 6 | 58,0548 | 6.7 | 256 |

### Charged Polymeric Carriers and Compositions Including Same

A composition as described herein that performs targeted transfection of cells expressing MHC class II (in particular APCs) for assessing the efficacy of a vaccine or other therapy or intervention or assessing the immunogenicity of a drug or biologic includes at least one charged (e.g., positively-charged) polymeric carrier such as a dendrimer having conjugated or bound thereto an MHC targeting molecule such as a universal DR binding peptide (e.g., an epitope such as tetanus toxin 582-599, the PADRE or Influenza HA T helper epitope: SFERFEIFPKEC (SEQ ID NO:28)) and at least one peptide, polypeptide or protein antigen, at least one allergen, at least one nucleic acid encoding the at least one antigen, at least one biologic, or at least one drug such that the at least one MHC targeting molecule and the at least one peptide or polypeptide antigen, at least one allergen, at least one nucleic acid encoding the at least one antigen, at least one biologic, or at least one drug are conjugated to the exterior surface of the charged (e.g., positively-charged) polymeric carrier (e.g., dendrimer) and the MHC targeting molecule specifically binds to PAPCs. In an embodiment in which the efficacy of a vaccine is being assessed, the combination of the at least one universal DR binding peptide, at least one dendrimer and at least one peptide or polypeptide antigen or at least one nucleic acid encoding the at least one antigen, are able to induce antigen presentation in APCs that results in stimulation of T cells specific for the antigen against which the vaccine was raised in PBMCs from an individual who has received the vaccine only if primed T cells specific for the antigen are present. By inducing activation of helper T cells specific for the antigen and cytokine expression, vaccine efficacy can be demonstrated. As described above, levels of the cytokines and/or extent of T cell proliferation/activation proportionally correlate with the T cell responses in the PBMCs from the individual. Thus, by measuring the levels of cytokines and extent of T cell proliferation/activation, whether or not a vaccine was effective in mounting an immune response against the antigen, and the extent of the immune response mounted, can be determined. ELISA, ELISpot and ICS in subjects with positive responses are generally 5-fold higher upon immunization that resulted in T cell responses. In intracellular cytokine assays (ICS), positive responses may range from about 0.02 to about 4% or more (e.g., 0.01, 0.05, 0.1, 0.5, 1.0, 1.5, 2.0, 2.5, 3.0, 3.5, 4.0, 4.5 %, etc.). IFNγ levels in an ELISA or Luminex/Multiplex assay in responders (i.e., a subject who has mounted an immune response against a vaccine) may be above 200 picograms. The number of positive spots may range from, for example, 50 to a few thousand spots per million in an ELISpot assay.

In an embodiment in which the immunogenicity of a drug or biologic or allergen is being assessed, the combination of the at least one universal DR binding peptide (e.g., T helper peptide), at least one dendrimer and at least one drug or allergen or biologic are able to induce cytokine production and proliferation and activation of T cells specific for the drug, allergen, or biologic. As described above, in a method of assessing the immunogenicity of a drug, allergen or biologic, if elevated cytokine levels and/or elevated levels or activation of T cells or B cells specific for the drug, allergen or biologic are detected in an assay as described herein, the drug, allergen or biologic is determined to be immunotoxic, because the drug, allergen or biologic has caused a T cell or B cell response in the subject(s).

Antigen or antigens as described herein that are displayed on or within the dendrimers resulting in activation and proliferation of T helper cells are used to detect an immune response mounted as a result of vaccination against antigen from any pathogen. In one embodiment, the antigen may be derived from, but not limited to, pathogenic bacterial, fungal, or viral organisms, *Streptococcus* species, *Candida* species, *Brucella* species, *Salmonella* species, *Shigella* species, *Pseudomonas* species, *Bordetella* species, *Clostridium* species, Norwalk virus, *Bacillus anthracis, Mycobacterium tuberculosis,* human immunodeficiency virus (HIV), *Chlamydia* species, human Papillomaviruses, Influenza virus, Paramyxovirus species, Herpes virus, Cytomegalovirus, Varicella-Zoster virus, Epstein-Barr virus, Hepatitis viruses, *Plasmodium* species, *Trichomonas* species, sexually transmitted disease agents, viral encephalitis agents, protozoan disease agents, fungal disease agents, bacterial disease agents, cancer cells, or mixtures thereof.

In one embodiment, the at least one universal DR binding peptide is two (e.g., two, three, four, five, etc.) universal DR binding peptides, each having the amino acid sequence of SEQ ID NO:1. In another embodiment, the at least one universal DR binding peptide is Influenza HA T helper epitope (SFERFEIFPKEC) (SEQ ID NO:28) or any of the peptides mentioned herein, e.g., those in Table 1. The universal DR binding peptide (MHC class II binding ligand) is any epitope or small molecule that specifically binds to MHC class II. Binding to MHC class II is required for the APC targeting ability of the compositions described herein, enabling delivery, expression, processing and presentation of the antigen which in turn is needed for the assessment of a vaccine's efficacy against a particular pathogen or antigen or for the assessment of unwanted T cell responses against drugs and/or biologics and/or cells. In an embodiment in which the dendrimer is conjugated to a nucleic acid encoding an antigen, the nucleic acid is generally an expression vector. The expression vector typically includes a eukaryotic promoter operably linked to a gene encoding the antigen, a cloning site, a polyadenylation sequence, a selectable marker and a bacterial origin of replication. Such plasmids bind naturally to the positively-charged derivatized dendrimers described herein. Generally, the antigen is typically a cancer antigen or an antigen from an infectious pathogen. The at least one dendrimer is generally a G5 dendrimer. Dendrimers are effective vehicles to escort DNA (and other nucleic acids including DNA, RNA, siRNA, microRNA, RNAi, etc.) into cells. Similarly, in embodiments in which the dendrimer is conjugated to a peptide or polypeptide antigen, the antigen is generally a cancer antigen or an antigen from an infectious pathogen, and the at least one dendrimer is a G5 dendrimer. In one embodiment of a composition for assessing the efficacy of a vaccine or for assessing the immunogenicity of a drug or biologic (e.g., antibody, cells, etc.), the universal DR binding peptide is a PADRE epitope and the dendrimer is PADRE-derivatized. PADRE is an artificially designed peptide that binds to the majority of MHC Class II, and conjugating PADRE peptides to dendrimers (e.g., a PADRE-derivatized dendrimer) makes the resultant complex or conjugate a ligand for PAPCs that express high levels of MHC class II. This complex thus becomes a universal targeted antigen delivery system with high affinity for cells expressing MHC class II or PAPCs. PADRE is a universal DR binding peptide that binds to many murine, non-human primates and human MHC class II molecules. It is a synthetic, non-natural T helper epitope [AKchxAVAAWTLKAAA (SEQ ID NO:26) (chxA = cyclohexylalanine)]. When fused to the surface of the dendrimer, PADRE will bind and activate primarily cells that have MHC class II including all PAPCs. Several PADRE epitopes (e.g., 2, 3, 4, 5, etc.) can be attached to each dendrimer. The attachment is done with suitable spacers to preserve the binding properties of the peptide that give rise to its MHC binding properties. A linker or spacer molecule may be used in conjugating antigen or other molecules to the dendrimer conjugates described herein. Spacers may be any combination of amino acids including AAA, KK, GS, GSGGGGS (SEQ ID NO:27), RS, or AAY. As used herein, the terms "linker" or "spacer" mean the chemical groups that are interposed between the dendrimer and the surface exposed molecule(s) such as the MHC class II ligand, CD4+ T helper epitope, polypeptide, or therapeutic agent that is conjugated or bound to the dendrimer (e.g., PADRE-dendrimer) and the surface exposed molecule(s). Preferably, linkers are conjugated to the surface molecule at one end and at their other end to the nanoparticle (e.g., PADRE-dendrimer). Linking may be performed with either homo- or heterobifunctional agents, i.e., SPDP, DSS, SIAB. Methods for linking are disclosed in PCT/DK00/00531 (WO 01/22995) to deJongh, et al.

Nucleic acid molecules encoding an antigen as described herein may be in the form of RNA (e.g., mRNA, microRNA, siRNA, shRNA or synthetic chemically modified RNA) or in the form of DNA (e.g., cDNA, genomic DNA, and synthetic DNA). The DNA may be doublestranded or single-stranded, and if single-stranded, may be the coding (sense) strand or noncoding (anti-sense) strand. In one embodiment, a nucleic acid can be an RNA molecule isolated or amplified from immortalized or primary tumor cell lines

As described above, in one embodiment, a composition for assessing the efficacy of a vaccine (an immune response mounted against the vaccine or antigen) includes at least one dendrimer having conjugated thereto at least one universal DR binding peptide and a nucleic acid encoding an antigen. The use of DNA for introducing an antigen in order to assess the efficacy of a vaccine or to assess the immunogenicity of a drug or biologic has several advantages. First, use of DNA provides a full spectrum of naive (naturally) processed epitopes. Also, dendrimers conjugated to a universal DR binding peptide and a nucleic acid encoding an antigen provide for targeted delivery to APCs of >95% of all human MHCs (AKA, HLA) and eliminate the need for the purification of proteins that are challenging to purify. Such proteins can be part of a multiprotein complex, can be membrane proteins, and can be incorrectly folded and insoluble. The dendrimer conjugates described herein do not require glycoslyation or posttranslational modifications of proteins, they tag interference with protein structure or folding, and offer dramatic cost and time savings. The fact that PADRE-dendrimer targets and delivers nucleic acids to PBMC from mice, Baboons and humans makes this platform an ideal candidate for rapid translational research from mice to non-human primates, and humans.

Also as described above, in another embodiment, a composition for assessing the efficacy of a vaccine (an immune response) or other therapy or intervention includes at least one dendrimer having conjugated thereto at least one universal DR binding peptide (e.g., an epitope such as the PADRE or Influenza HA T helper epitope: SFERFEIFPKEC (SEQ ID NO:28)) and a peptide or polypeptide antigen, wherein the at least one universal DR binding peptide and the peptide or polypeptide antigen are conjugated to the exterior surface of the dendrimer and are able to be taken up by APCs. Polypeptides and peptides with a negative net charge may complex with, for example, PADRE-dendrimer with no need for covalent conjugation.

As mentioned above, the compositions, kits, assays and methods described herein can be used to assess the efficacy of a vaccine or other therapy or intervention administered to a subject who is being treated for any infectious pathogen or cancer. Examples of infectious pathogens include viruses such as, but not limited to, influenza, HIV, dengue virus, rotavirus, HPV, HBV, HCV, CMV, HSV, HZV, and EBV, pathogenic agents including the causative agents of Malaria, *Plasmodium(p) falciparum, P. malariae, P. ovale, P. vivax* and *P. knowlesi;* the casatve agent of Leishmania (L), *L. major, L. tropica, L. aethiopica, L. mexicana, L. donovani, L. infantum* syn. *L. chagas;* pathogenic bacteria including *Bacillus anthracis, Bordetella pertussis, Streptococcus pneumonia,* and *meningococcus.*

Dendrimers conjugated to a universal DR binding petpide and an antigen or a nucleic acid encoding an antigen as described herein can be used to assess the efficacy of a vaccine against any cancer or any other therapy or intervention for cancer. Examples of cancers include HPV-induced cervical cancers (e.g., E7/E7 tumor associated antigens (TAA) or plasmids encoding for these antigens can be complexed with the universal DR binding peptide/dendrimers (e.g. PADRE-dendrimer) described herein), human melanoma (e.g., TRP-1, TRP-2, gp-100, MAGE-1, MAGE-3 and / or p53 may be used as TAA and complexed with the universal DR binding peptide/dendrimers (e.g. PADRE-dendrimer) described herein), and prostate cancer (e.g., TSA may be used as TAA and complexed with the universal DR binding peptide/dendrimers (e.g. PADRE-dendrimer) described herein). Similarly for lung tumors, breast tumors, and leukemia, any suitable TAA can be used, and many have been described. Many such TAA are common between various cancers (e.g., CEA, MUC-1, Her2, CD20).

### Methods and Assays for Detecting An Immune Response To a Vaccine or Other Therapy or Intervention

Described herein are assays, reagents, kits and methods for determining if a subject who has received a therapy or intervention (e.g., vaccination) for treating or preventing a pathology (e.g., infection) has mounted an immune response to the therapy or intervention as well as quantitating the immune response. These methods, reagents, kits and assays are useful for assessing the efficacy of a vaccine, for example. FIG. 11 illustrates a typical method, kit and assay for detecting an immune response to a vaccine or other therapy or intervention. Antigens or nucleic acids encoding the antigens are complexed with a peptide (e.g., PADRE)-derivatized-dendrimer and are used to transfect APCs in PBMC preparations, and convert them to antigen-expressing autologous APCs (referred to herein as "target cells").

In one example of a method and assay for detecting an immune response to a vaccine or other therapy or intervention, a first sample is obtained from a subject prior to the subject receiving the vaccine or other therapy or intervention, wherein the vaccine or other therapy or intervention includes an antigen, drug or biologic; a second sample is obtained from the subject after the subject has received the vaccine or other therapy or intervention; the first and second samples are each contacted with highly branched polymeric dendrimers conjugated to: an MHC targeting and universal DR binding peptide, and the antigen, drug or biologic; measuring an immune response in the first sample and measuring an immune response in the second sample; correlating an increased immune response in the second sample relative to an immune response in the first sample with an immune response against the vaccine or other therapy or intervention. In this method and assay, measuring an immune response typically includes measuring or detecting one or more of: T cell activation, T cell proliferation, B cell activation, B cell proliferation, and cytokine expression.

In another embodiment, determining if a subject who has received a therapy or intervention (e.g., vaccination) for treating or preventing a pathology (e.g., infection) has mounted an immune response to the therapy or intervention as well as quantitating the immune response does not involve obtaining a first sample from the subject prior to receiving the therapy or intervention (e.g., a vaccination). Such methods and assays can be useful, for example, in immunomonitoring techniques for tumor antigens in patients that have not received any vaccination (sometimes, immunity is spontaneously is primed even in the absence of a vaccine). In this embodiment, one or more irrelevant antigens (i.e., an antigen that is not included in the vaccine or related to the therapy or intervention) that individuals are not expected to have been exposed to are used as negative controls. An example of such a method for determining the efficacy of a vaccine includes the following steps. PBMCs obtained from the subject (e.g., a sample including PBMCs is obtained from the subject) after the subject has been vaccinated are divided into at least three portions (one may want to have more positive and negative controls) and placed in different wells (e.g., on a multi-well plate). Each portion includes approximately five million cells. One portion, the "effector cells," receives no treatment and is incubated in regular media in an incubator (37°C/5% CO₂). This portion is called specimen A in this example. To the other portion, called specimen B in this example, PDD complexed with the vaccine antigen or with a nucleic acid encoding the vaccine antigen is added. To a third portion, called specimen C in this example, PDD complexed with the irrelevant protein or peptide (or to a nucleic acid encoding the irrelevant protein or peptide) are added. These PDD serve as a negative control. Examples of irrelevant (control) proteins include albumin and Firefly Luciferase proteins. As another negative control, PDD that are not complexed to an irrelevant protein or peptide or to the antigen can be added to PBMC. As another negative control, the plasmids with no insert (empty vectors) complexed with PDD may be added to PBMC. Optional positive controls such as recall antigens such as those against Tetanus Toxoid or Influenza antigens (protein or plasmids encoding proteins) complexed to dendrimers as described herein may also be used in additional wells. After overnight (or other suitable amount of time) incubation in an incubator (37°C/5% CO₂), the specimen A is mixed separately with specimen B and C. The T cell responses will be measured upon 12-48 hours via optional methods such as ELISA for the measurement of IFN-γ in the supernatants of the test (mixture of A and B), and the negative control (mixture of A and C). A statistical difference, typically a five-fold difference, demonstrates a positive T cell response to antigen tested versus the negative control.

In this embodiment, a typical method of detecting an immune response to a vaccine or other therapy or intervention includes the following steps. A first composition including a plurality of charged highly branched polymeric dendrimers each having conjugated thereto at least one universal DR binding peptide and at least one peptide or polypeptide antigen or a nucleic acid encoding the at least one antigen is prepared or provided, the at least one universal DR binding peptide and the nucleic acid or at least one peptide or polyeptide antigen being conjugated to the exterior surface of the plurality of charged highly branched polymeric dendrimers such that the at least one universal DR binding peptide specifically binds to PAPCs. In this embodiment, the vaccine or other therapeutic intervention includes the antigen or a portion thereof. A first sample including PAPCs from the subject is obtained after the subject has been vaccinated, and the first sample is divided into at least a first portion and a second portion. The at least first portion is contacted with the first composition under incubation conditions such that the plurality of charged highly branched polymeric dendrimers are taken up by the PAPCs and such that the antigen is processed by the PAPCs and presented by the PAPCs in combination with MHC class II. The second portion is contacted with a second composition that includes a plurality of charged highly branched polymeric dendrimers each having conjugated thereto at least one universal DR binding peptide and at least one negative control peptide or polypeptide or a nucleic acid encoding the at least one negative control peptide or polypeptide, the at least one universal DR binding peptide and the at least one control peptide or polyeptide antigen or nucleic acid encoding the at least one negative control peptide or polypeptide being conjugated to the exterior surface of the plurality of charged highly branched polymeric dendrimers such that the at least one universal DR binding peptide specifically binds to PAPCs. The at least first portion contacted with the first composition is examined for the presence of at least one molecule or marker that is indicative of an immune response to the vaccine or other therapeutic intervention, and the level of the at least one molecule or marker is determined. Similarly, the at least second portion contacted with the second composition is examined for the presence of the at least one molecule or marker, and the level of the at least one molecule or marker is determined. The level of the at least one molecule or marker in the at least first portion contacted with the first composition is compared with the level of the at least one molecule or marker in the at least second portion contacted with the second composition, and a higher level of the at least one molecular or marker in the at least first portion contacted with the first composition than in the at least second portion contacted with the second composition is correlated with an immune response to the vaccine. If a higher level of the at least one molecular or marker is not detected or measured in the at least first portion contacted with the first composition relative to the level in the at least second portion contacted with the second composition, it is not concluded that an immune response was mounted against the vaccine or other therapeutic intervention. The at least one control peptide or polyeptide antigen can be any suitable negative control, such as for example, albumin or luciferase. The at least one molecule or marker that is indicative of an immune response to the vaccine or other therapeutic intervention can be, for example, T cell or B cell activation or proliferation, or a cytokine (e.g., IFN-γ). Examining the at least first portion contacted with the first composition and the at least second portion contacted with the second composition for the presence of the at least one molecule or marker, and determining the level of the at least one molecule or marker in the at least first portion contacted with the first composition and the at least second portion contacted with the second composition can be performed using any suitable assay, such as a cytokine assay and/or CTL assay.

An example of an application in which this embodiment may be particularly useful relates to the unsuccessful search for detecting and/or discriminating Active versus Latent tuberculosis (TB). The T SPOT.TB test is an *in vitro* diagnostic test that measures T cells specific to Mycobacterium tuberculosis (MTB) antigens. This test involves use of a cocktail of peptide epitopes, and it is positive even after treatment due to memory T cells to immunodominant peptides that are used in the test. In order to discriminate Active vs. Latent TB using the methods, kits, reagents and assays described herein, one can use a plasmid encoding antigens that are specific for Latent TB infection with PDD as described herein and measure IFN levels.

In the methods, any suitable amount of a composition including autologous APCs obtained as described above effective to induce MHC class II-mediated activation of helper T cells is used. One example is to use 1 million PBMCs in 2 ml (relevant) media in a well in a 24-well plate. 1-5 ug of plasmid DNA or antigen or 0.5ug siRNA mixed with PDD in a ratio of (4-10):1, where 4-10 fold PDD is used. The at least one universal DR binding peptide can be two Pan-DR epitopes each having the amino acid sequence of SEQ ID NO:1. Alternatively, the at least one universal DR binding peptide can be other than a Pan-DR epitope (PADRE epitope), e.g., influenza HA. Generally, the at least one dendrimer is a G5 dendrimer. A human patient who is a candidate for receiving a particular biologic or drug, or who has been vaccinated or received another therapy or intervention, or will be vaccinated or receive another therapy or intervention, or who is being considered for vaccination or other therapy or intervention is a typical subject.

### Methods and Assays for Assessing the Immunogenicity of a Drug or Biologic

Further described herein are methods, reagents, kits and assays for assessing immunogenicity of a drug or biologic. These methods, reagents and assays can be used to assess the immunogenicity (or lack thereof) of any drug or biologic. A typical method of assessing whether or not a drug or biologic is immunogenic in a subject or a population of subjects includes the following steps: obtaining a sample including PBMCs from a subject; dividing the sample into at least a first portion, a second portion, and a third portion; adding to the second portion a composition including a plurality of charged highly branched polymeric dendrimers each having conjugated thereto at least one universal DR binding peptide and at least one drug or biologic, wherein the at least one universal DR binding peptide and the drug or biologic are conjugated to the exterior surface of the plurality of charged highly branched polymeric dendrimers such that the at least one universal DR binding peptide specifically binds to PAPCs; adding to the third portion a composition including a plurality of charged highly branched polymeric dendrimers each having conjugated thereto at least one universal DR binding peptide and at least one negative control polypeptide or nucleic acid encoding the negative control polypeptide, wherein the at least one universal DR binding peptide and the negative control polypeptide or nucleic acid encoding the negative control polypeptide are conjugated to the exterior surface of the plurality of charged highly branched polymeric dendrimers such that the at least one universal DR binding peptide specifically binds to PAPCs; incubating the at least first portion, second portion and third portion; mixing a first aliquot of the first portion with the second portion or an aliquot thereof, mixing a second aliquot of the first portion with the third portion or aliquot thereof, and incubating the mixtures under conditions that allow T cell proliferation and activation; measuring T cell proliferation or activation in the mixture of the first aliquot of the first portion with the second portion; measuring T cell proliferation or activation in the mixture of the second aliquot of the first portion with the third portion; and correlating an increased T cell proliferation or activation in the mixture of the first aliquot of the first portion and the second portion compared to T cell proliferation or activation in the mixture of the second aliquot of the first portion and the third portion with an immune response to the drug or biologic. In the method, measuring T cell proliferation or activation includes measuring IFN-γ levels, or the level(s) of any other cytokine. Also in the method, an immune response to the drug or biologic is typically correlated with immunogenicity of the drug or biologic.

One example of a method and assay for assessing the immunogenicity of a drug or biologic includes the following steps. PBMCs obtained from the subject(s) or populations to be tested for adverse immune reactions are divided into at least three portions (one may want to have more positive and negative controls) and placed in different wells (e.g., on a multi-well plate). Each portion includes approximately five million cells. One portion, the "effector cells," receives no treatment and is incubated in regular media in an incubator (37°C/5% CO₂). This portion can be called specimen A. To a second portion, specimen B, is added PDD complexed with the biologics/drug/compound or self antigens or with a nucleic acid encoding a biologic or self-antigen are added. To a third portion, called specimen C, PDD complexed with the irrelevant protein or peptide (or to a nucleic acid encoding the irrelevant protein or peptide, or empty plasmid) are added. These PDD serve as a negative control. Examples of irrelevant (control) proteins include albumin and Firefly Luciferase proteins. As another negative control, PDD that are not complexed to an irrelevant protein or peptide or to the antigen can be added to PBMCs. As another negative control, the plasmids with no insert complexed with PDD may be added to PBMCs. Optional positive controls such as recall antigens such as those against Tetanus Toxoid or Influenza antigens (protein or plasmids encoding proteins) complexed to dendrimers as described herein may also be used in additional wells. After overnight (or proper time) incubation in an incubator (37°C/5% C_{O2}), the specimen A is mixed separately with specimens B and C. The T cell responses are measured upon 12-48 hours via optional methods such as ELISA for the measurement of IFN-γ in the supernatants of the test (mixture of A and B), and the negative control (mixture of A and C). A statistical difference, typically a five-fold difference, demonstrates a positive T cell response to antigen tested versus the negative control. Regarding the preparation of PDD/antigen/drug complex, such dendrimer/DNA plasmid complex or dendrimer/biologic or drug complexes are prepared as described herein. Different ratios of PDD to DNA plasmid or biologic or drug are tested. Gel electrophoresis can be performed as a standard assay to determine the best ratio that results in retention of the cargo in the gel. One example of a ratio is 7 times (by weight) PDD and one time drug/protein, biologic, RNA or plasmid DNA. 2-5 µg of either of drug, protein, biologic, or RNA or plasmid DNA in a final volume of 100 µl in PBS or OptiMem can be used. To make the complex, 100 µl for each reaction is used. This is typically left at room temperature for approximately 10 minutes. 100 µl of the complex is added to one million PBMCs in 2 ml of RPMI media. This mixture is cultured in a 37° C/5% _{CO2} incubator overnight. One million PBMCs of the same individual or animal are added, and incubated in a 37° C/5% _{CO2} incubator for 24 hours. Cytokine levels and/or T cell proliferation are measured by any desired method.

### Compositions and Methods for Delivering a Nucleic Acid To A Cell

In the experiments described herein, delivery of a gene encoding GFP was specifically delivered to MHC Class II cells (cells expressing MHC Class II) and expression of the gene was observed. Thus, the compositions and methods described herein may find use in any gene therapy application. A composition for delivering a nucleic acid to a cell typically includes at least one positively-charged highly branched polymeric dendrimer having conjugated thereto at least one universal DR binding peptide (e.g., T helper peptide) and at least one nucleic acid encoding a peptide or protein, wherein the at least one universal DR binding peptide and the nucleic acid are conjugated to the exterior surface of the at least one positively-charged highly branched polymeric dendrimer such that the at least one universal DR binding peptide specifically binds to the cell, and the combination of the at least one universal DR binding peptide, at least one positively-charged highly branched polymeric dendrimer, and the nucleic acid are internalized by the cell. A method of delivering a nucleic acid to a cell typically includes contacting the cell with a composition including at least one positively-charged highly branched polymeric dendrimer having conjugated thereto at least one universal DR binding peptide and at least one nucleic acid encoding a peptide or protein, wherein the at least one universal DR binding peptide and the nucleic acid are conjugated to the exterior surface of the at least one positively-charged highly branched polymeric dendrimer such that the at least one universal DR binding peptide specifically binds to the cell, and the combination of the at least one universal DR binding peptide, at least one positively-charged highly branched polymeric dendrimer, and the nucleic acid are internalized by the cell. In a typical embodiment, the peptide or protein is expressed within the cell.

One example of a method of delivering a nucleic acid into professional antigen presenting cells includes the steps of: providing a composition including at least one charged highly branched polymeric dendrimer having conjugated thereto at least one Class II-associated invariant chain peptide (CLIP), wherein the at least one CLIP is conjugated to the exterior surface of the charged highly branched polymeric dendrimer such that the at least one CLIP specifically binds to professional antigen presenting cells; and contacting the composition with a plurality of cells from a mammalian subject (e.g., human) under conditions in which the at least one charged highly branched polymeric dendrimer having conjugated thereto at least one CLIP binds to a professional antigen presenting cell within the plurality of cells. In some embodiments, the charged highly branched polymeric dendrimer is a PAMAM dendrimer and the at least one CLIP is CLIP p88-99. The charged highly branched polymeric dendrimer can be further conjugated to at least one nucleic acid (e.g., siRNA, microRNA, RNAi, RNA or DNA), and the nucleic acid enters the professional antigen presenting cell. The at least one charged highly branched polymeric dendrimer can include a drug (e.g., carry and deliver a drug).

### Kits for Assessing Efficacy of A Vaccine or Other Therapy or Intervention and for Assessing Immunogenicity of A Drug or Biologic

Also described herein are kits for assessing the efficacy of a vaccine or other therapy or intervention and for assessing the immunogenicity of a drug or biologic. FIG. 11 illustrates how a typical kit is used for assessing the efficacy of a vaccine or other therapy or intervention or for assessing the immunogenicity of a drug or biologic. A typical kit includes a container that includes a plurality of dendrimer/universal DR binding peptide complexes (conjugates) as described herein (e.g., PADRE-derivatized dendrimers, dendrimers conjugated to influenza HA, etc.), a physiological buffer, and instructions for use. The dendrimer/universal DR binding peptide complexes can be conjugated to nucleic acids, peptide, proteins, drugs, or biologics, depending on the intended use. Because of the universal nature of the kit, it can be purchased and used to assess the efficacy of any vaccine or other therapy or intervention, and/or to assess the immunogenicity of any biologic or drug. Typically, a buffer with a pH of 7.4 is used to dilute PDD, DNA, RNA, or antigen. In one example of a buffer or medium, the buffer or medium includes Eagle's Minimal Essential Medium, buffered with HEPES and sodium bicarbonate, and supplemented with hypoxanthine, thymidine, sodium pyruvate, L-glutamine, and less than 10% serum bovine albumin or individual serum proteins including insulin and/or transferrin with 100 mg/L CaCl₂ where the endotoxin level is less than 1.0 EU/mL. In other embodiments, the buffer or media used to make the complex of PDD (peptide-derivatized-dendrimer) is a water-based salt solution containing sodium chloride, sodium phosphate, and (in some formulations) potassium chloride and potassium phosphate such as PBS (phosphate Buffered Saline).

In an embodiment in which a nucleic acid is to be complexed with the dendrimer/universal DR binding peptide complexes, a user of the kit dilutes at least one nucleic acid (e.g., DNA plasmid) encoding one antigen with the buffer at 100-200 µg /ml, and while shaking gently, adds the composition (universal DR binding peptide-dendrimer) to the diluted plasmid DNA. In a typical embodiment, a ratio of 10:1 of universal DR binding peptide-dendrimer to plasmid DNA is used (N:P), which is approximately 7 times (weight) of composition to one time (weight) of DNA plasmid(s).

In an embodiment in which the dendrimer/universal DR binding peptide complexes are conjugated to proteins or polypeptides or allergens, typically the same ratio of 10:1 of the dendrimer/universal DR binding peptide complexes to protein or allergen results in the complex formation. The instructions for use included in a kit as described herein describes the protocol of making proper ratios, buffers, and optimization and troubleshooting when needed. Complexation of plasmid DNA, protein/antigen, allergen, drug, or biologic with the universal DR binding peptide-dendrimers (e.g., PADRE-dendrimer conjugates) described herein is generally done by mixing the two components in aqueous solution buffered at physiological pH with a physiological buffer including PBS. Typical N/P (amine to phosphate) ratios are 10:1. Gel electrophoresis or other suitable assay can be used to demonstrate complete complexation of the DNA to the universal DR binding peptide-dendrimers (e.g., PADRE-dendrimer conjugates).

In some embodiments, a kit as described herein may be used to predict unwanted (undesired) T cell and/or B cell responses against all kinds of chemicals and compounds that are used in drugs, biologics or other consumable or make up reagents used as preservatives, to enhance stability, or to increase delivery where a T cell and/or B cell response may be responsible or partly involved. For example, a Delayed-type hypersensitivity (DTH) is a cell-mediated immune memory response, or Type 4 hypersensitivity where an immunologically mediated reaction involving sensitized T cells mediate delayed type hypersensitivity reactions resulting in allergic contact dermatitis. Type 1 (IgE related) and Type 4 reactions (T cell related) may occur in the same individual. The development of contact dermatitis may precede the onset of IgE mediated symptoms. DTH reactions can be very severe and associated with significant morbidity.

A kit as described herein can be used with any antigen, allergen, drug, biologic, nucleic acid sequence, vector or plasmid encoding an antigen of interest. Instructional materials for preparation and use of the dendrimer/universal DR binding peptide complexes (conjugates) described herein are generally included. While the instructional materials typically include written or printed materials, they are not limited to such. Any medium capable of storing such instructions and communicating them to an end user is encompassed by the kits and methods herein. Such media include, but are not limited to electronic storage media (e.g., magnetic discs, tapes, cartridges, chips), optical media (e.g., CD ROM), and the like. Such media may include addresses to internet sites that provide such instructional materials.

### EXAMPLES

The present invention is further illustrated by the following specific examples. The examples are provided for illustration only and should not be construed as limiting the scope of the invention in any way.

### Example 1 - An adjuvanted/targeted Nanoparticle-based Platform for Producing Autologous APCs Presenting Antigen

The dendrimer-based nanoparticles described herein are typically prepared by the conjugation of two reactants: a fifth-generation, amino-terminated, PAMAM dendrimer, and a targeting/immune-enhancing peptide, or universal DR binding peptide (e.g., PADRE). The data described below showed this platform increase transfection efficiency in both mouse and human APCs by 2- to 3-fold. Moreover, *in vivo* experiments using GFP-encoding plasmid conjugated to PADRE-dendrimer showed that GFP is produced in the draining lymph nodes.

### Materials and Methods

PADRE-derivatized PAMAM dendrimer was generated as described above with the following modifications. The PADRE-dendrimer/DNA or siRNA complex was generated by incubation at room temperature for 10 minutes at a proper N/P ratio. Such complexes were added to primary PBMC or splenocytes for *in vitro* studies or injected subcutaneously for vaccination purposes. FIG. 1 shows PADRE decoration of (conjugation to) fifth-generation PAMAM Dendrimer.

To maintain the highly positively-charged surface for binding of multiple nucleic acids, one dendrimer molecule typically has two PADRE peptides conjugated to its surface so that it will still keep its positive net charge. Addition of PADRE to the dendrimers results in specific targeting of APCs, and strong CD4 help.

### Results

The prepared PADRE-dendrimers were characterized. The peptide-dendrimer conjugate was made by simple amide coupling between the -COOH terminus of the peptide and the dendrimer amine groups. A 2:1 peptide/dendrimer challenge ratio was used in the reaction, seeking attachment of just a few peptides per dendrimer, in order to keep most of the free amine groups to develop large positive charges on the dendrimer. The product was purified by dialysis against pure water for at least 24 h and then dried under vacuum. The collected product, a clear oil, was characterized by 1H NMR, UV-Vis and MALDI-TOF mass spectroscopy. NMR shows large peaks corresponding to the dendrimer protons and a small set of peaks for the peptide protons. The MALDI-TOF mass spectrum of the PADRE- dendrimer conjugate shows a peak at a m/z ratio ca. 3,000 units higher than the peak observed for the dendrimer on its own. The excess mass corresponds to approximately 2 peptide epitopes.

The data established that an average of two PADRE are present on each dendrimer. *In vitro* delivery of multiple nucleic acids into autologous APCs was shown. *In vitro* multinucleotide delivery/transfection of human primary peripheral mononuclear cells was best achieved in the charge ratios of 1:5 and 1:10. FIG. 2 shows dsRNA delivery (~ %86) via PADRE-dendrimers into purified human B cells where Alexa Fluor-tagged dsRNA complexed with (conjugated to) PADRE-dendrimer was incubated with B cells for 4 hours. Cells were stained with CD19/FITC and the red channel (PE) represents cells with the dsRNA /Alexa Fluor. man

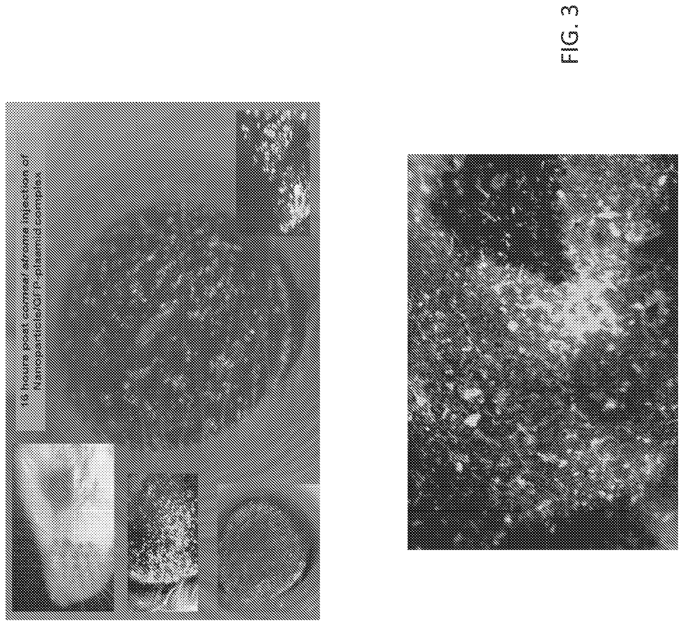

Referring to FIG. 3, *in vivo* DNA delivery of PADRE-dendrimers was shown. Plasmids encoding GFP or TRP-2 were injected alone or complexed with PADRE-dendrimer, or dendrimer (i.e., dendrimer not complexed with PADRE). The images show the expression of GFP in skin (left) and cornea (right) 24 and 16 hours post-injection. Effective expression of GFP is demonstrated in both skin and cornea 24 and 16 hours post-injection of PADRE-dendrimer complexes. Targeting of the lymph nodes *in vivo* was demonstrated. Eight days after PADRE-dendrimer/GFP-plasmid complexes were injected subcutaneously (5µg total plasmid), the adjacent lymph node was removed and compared with lymph nodes of a mouse injected with GFP-DNA alone. Fluorescent microscope images were taken on meshed lymph nodes on day eight post-immunization. Expression of antigen in the lymph node adjacent to the injection site was seen, but expression of antigen in a control lymph node was not seen.

Targeting of the lymph nodes *in vivo* was demonstrated. Eight days after PADRE-dendrimer/GFP-plasmid complexes were injected subcutaneously (5µg total plasmid), the adjacent lymph node was removed and compared with lymph nodes of a mouse injected with GFP-DNA alone. Fluorescent microscope images were taken on meshed lymph nodes on day eight post-immunization. Expression of antigen in the lymph node adjacent to the injection site was seen, but expression of antigen in a control lymph node was not seen.

These data clearly demonstrate that the targeted adjuvanted nanopatricle platform described herein results in gene delivery, robust expression of the encoded antigen, and antigen presentation. Thus, the PADRE-dendrimer nanoparticles described herein are a novel and powerful adjuvanted/targeted delivery tool and platform for delivery of dsRNA.

### Example 2 ―In vitro Targeted Delivery and Transfection Efficiency

Referring to FIG. 4, *in vitro* targeted delivery of PBMCs results in 77% B cell transfection efficiency. Human PBMC from healthy donors were obtained. PBMCs were cultured at 6 million cells per ml of RPMI media with 10% fetal bovine serum. The plasmid encoding for GFP at 5 µg was diluted in 100 ul of a physiological buffer, PBS, and 5 µg of PADRE-dendrimer in 50 ul PBS was added to DNA while shaking. After 10 minutes incubation at room temperature, the mixture/complex of the GFP plasmid and PADRE-dendrimer was then added to PBMC. Twenty-four hours post incubation at 37 °C / 5% CO₂ incubator, PBMCs were stained with CD19 PE and cells were analyzed by flow cytometry. The expression of GFP was observed in 43% of total PBMC while when gated on B cells 77% of B cells expressed GFP. Control groups, PBMC incubated with same ratios of dendrimer and GFP plasmid showed about 11% and 7% GFP expression in total PBMC or B cells. No major viability change was observed when compared with PBMC with only media. This is a representative experiment of several. These experiments demonstrate i) the delivery of GFP plasmid into PBMC and in particular to MHC class II expressing cells (B cells), and ii) the expression of the GFP by PBMC and in particular by B cells.

### Example 3 - Delivery of Peptides/Proteins Into Mouse DCs In Vivo and Human B cells In Vitro

PDD/Albumin-FITC was delivered into purified human B cells (FIG. 5). Referring to FIG. 6, this Figure shows PADRE-dendrimer targeting of and efficacy in mouse DCs *in vivo* and a timeline for injection and lymph node analysis. The results of this experiment show that i) (FIG. 5) Albumin-FITC, a protein, mixed with PADRE-dendrimer was delivered in human B cells in less than two hours, ii) (FIG. 6) in day 5 post subcutaneous injection, PADRE, an epitope, conjugated to dendrimer was delivered into lymph node's B cells and DCs *in vivo* (the PADRE-dendrimer was complexed to GFP-plasmid to visualize the delivery of the complex to lymph node/B cells/DCs.), iii) (FIG. 7) in day 5 post subcutaneous injection, HA helper epitope of influenza, an epitope, conjugated to dendrimer was delivered into lymph node's DCs *in vivo* (the PADRE-dendrimer was complexed to GFP-plasmid to visualize the delivery of the complex to lymph node DCs). These data were representative of several experiments and in some the lymph nodes were removed on day 3 post subcutaneous injection of PADRE-dendrimer or HA-dendrimer each complexed with GFP plasmid. These results establish examples of the delivery to APCs including B cells and DC of a protein conjugated with FITC via FITC visualization of FITC as well as the delivery of two peptides, PADRE and HA helper epitopes conjugated to dendrimer where GFP plasmid was complexed with the peptide-dendrimer to facilitate visualization and analysis of the complex (peptide-linked to dendrimer complex with GFP-encoding plasmid) in the cells of lymph nodes.

Specific *in vitro* and *in vivo* transfection of DCs was shown by *in vivo* flow cytometry data on targeting and expanding DCs in an adjacent lymph node, 5-days post-injection of the nanoparticle (PADRE-dendrimer/GFP-encoding plasmid) vs. controls (78% vs. ~7% GFP expression). The PADRE-derivatized dendrimer (PDD) enhances delivery due to its assisted opsonized effect of PADRE which with high affinity binds to MHC class II expressed on APC. Similarly, HA-dendrimer (DRHA)/GFP-plasmid was delivered *in vivo* in the neighboring lymph nodes, when injected subcutaneously (FIG. 7). Note that in mice, PADRE binds the MHC class II of IAb (C57BL mice) (FIG. 6) while selected HA epitope binds the MHC class II of IAd (Balb/c mice) (FIG. 7). The feasibility *of in vivo* delivery in two different mice strains with two different epitopes with similar results have been shown. The APC-targeted delivery resulting in the expression of GFP by PADRE-dendrimer/GFP-plasmid into human PBMCs (FIG. 4), purified human B cells (FIG. 4), and in splenocytes of C57BL mice, and the delivery of PADRE-dendrimer/dsRNA into human B cells (FIG. 8) and of monkey PBMC (FIG. 9) are additional *in vitro* evidence of the delivery of peptide to PAPCs by the compositions described herein. Because use of two different targeting peptides, whose unique feature is to bind to the MHC class II, works as shown in the experiments described herein, the methods, kits, assays and compositions described herein encompass all MHC class II binding peptides. Referring to FIG. 7, dendrimer conjugated to influenza HA helper epitope (HDD) was also prepared.

### Example 4 - PADRE-Dendrimer Delivery of dsRNA Into Human B Cells and Non-human Primate PBMCs and PADRE-Dendrimer Delivery of Plasmid Into Non-human Primate PBMCs

Referring to FIG. 8, PADRE-dendrimers complexed to dsRNA (a nucleic acid) exhibited targeted delivery *in vitro.* 0.1 µg of dsRNA was diluted in 100 ul of PBS and 0.7 µg of the PADRE-dendrimer in 20 ul was added to dsRNA-Alexa Fluor tagged while shaking. The complex, after a 10 minute incubation at room temperature, was added to one million purified B cells (in RPMI plus 10% fetal bovine serum) in wells of a 24-well plate. About an hour post incubation at 37° C/5% CO₂ incubator, cells were washed and placed back in the wells (in 1 ml of fresh RPMI plus 10% fetal bovine serum) and were analyzed under fluorescent microscope in red channel. The overlay image of cells under bright field and red channel demonstrates the uptake of Alexa Fluor tagged dsRNA by human B cells (FIG. 8). Cells were incubated overnight at 37° C/5% CO₂ incubator when they were stained with CD19 (a B-cells marker) and analysed by flow cytometry (FIG. 2). As shown in the FIG. 2, >80% of the B cells were positive for Alexa Fluor (tagged to dsRNA) versus about 6% for the control, dendrimer/dsRNA-Alexa Fluor. These results clearly demonstrate the robust delivery of nucleic acids to PAPC by PADRE-dendrimer.

PBMCs one sample from baboon (papio hamadryas), and two different samples from cynomolgus monkeys (macaca fascicularis) were tested. Fluorescent microscope images shown in FIG. 9 are representative, taken two hours post-addition of PADRE-dendrimer or dendrimer, each complexed with dsRNA/Alexa Fluor. Similarly, PADRE-dendrimer or dendrimer complexed with GFP-plasmid were added to the PBMCs and were analyzed 24 hours after incubation (FIG. 10). The results show that, in less than 2 hours, PADRE-dendrimer delivers nucleic acids into the monkeys' PBMCs, while dendrimer shows only a modest delivery. These results strongly suggest that PADRE-dendrimer works on non-human primates.

### Example 5 - Evaluation of Cell-mediated Immune Responses to Vaccination

The assays, kits, compositions and methods described herein provide several advantages. These advantages include: a full spectrum of native/naturally processed epitopes becomes available for immunoevaluation on the cells from the same individual; EBV infection of PBMCs, infecting stimulated cells by viral vectors, using CD40 expressing cells, peptide loading of cells are labor intensive/expensive and not feasible for unspecialized laboratories in all sites; unlike current methods of DNA delivery that are not efficient and induce poor immune responses, the methods described herein result in strong antibody responses that implies high expression; and the methods described herein are rapid. In addition, viral delivery of genes to PBMCs results in strong immune responses to viral vectors and handling viral vectors is associated with safety concerns.

In a method of assessing the efficacy of a vaccine, a dendrimer tagged with PADRE makes a complex with antigen, DNA or RNA that encodes the vaccine antigen in 10 min (such complexes were demonstrated by electrophoresis). This complex is co-cultured with prevaccinated PBMC (overnight) and used to transfect APCs. Such cells are treated with mitomicin-C and are used as target cells (autologous APCs) expressing the antigen that was used in vaccination. The PADRE on the surface of dendrimer targets MHC class II on APCs.

The transfection of primary CD3 T cells among human PBMCs was demonstrated. Ten ug of GFP-plasmid was complexed with a nanoparticle as described herein for 10 minutes. The complex of nanoparticle and DNA was co-cultured overnight with PBMCs. A FACS analysis was performed on days 3 and 7 post-transfection. The transfection of primary purified CD19 B cells was also demonstrated, establishing the feasibility of transfection of APCs via co-culturing with nanoparticle/GFP-plasmid. Ten ug of GFP-plasmid was complexed with the nanoparticle for 10 minutes. The complex of nanoparticle and DNA was co-cultured overnight with purified human B cells, and the FACS analysis was performed on day 2 post-transfection.

Referring to FIGS. 15-17, the experimental results shown in these figures evidence the usefulness of the dendrimers described herein for delivery of nucleic acids and peptides/polypeptides and for assessing vaccine efficacy in mammals. In FIG. 15, high levels of delivery (73%) of PDD/albumin-FITC in human B cells clearly shows that the platform may be used with proteins/polypeptides or their like antigens. In FIG. 16, the high levels of delivery (77%) of PDD/GFP plasmid in human B cells clearly shows that the platform efficiently delivers plasmids into B cells and results in the expression of encoded protein/antigen. FIG. 17 shows that vaccination efficacy was measured in mice; note the significant differences in the levels of IFN-γ in vaccinated mice.

FIG. 14 is a photograph of results showing A) UV spectra of dendrimer, PADRE and dendrimer-PADRE. UV spectra of peptide-dendrimer was performed by standard methods. The phenylalanine peak seen for G5 dendrimer-PEDRE shows that the peptide, PADRE, is added to the dendrimer. B) Agarose gel electrophoresis and electrophoretic mobility analysis of dendrimer/DNA complex. Analysis of the complex formation of and the binding of PDD to DNA was performed by examining the retardation in the migration of the plasmid DNA during agarose gel electrophoresis. Peptide-derivatized-dendrimer (PDD)/plasmid complexes were tested for their retainment of DNA in gel electrophoresis. Gel electrophoresis was performed for PDD/plasmid and controls: DNA alone, dendrimer alone, and [PDD/plasmid] samples where various ratios, 1:1, 1:2, 1:5, 1:10, 1:20 of (P:N). The PDD was able to retain DNA plasmid in ratios > (1:2). FIG. 15 is a series of flow cytometry Dot Plot diagram showing cell specific delivery of proteins/antigens. PDD/albumin-FITC was delivered into purified human B cells. PBMC were co-cultured with either of albumin-FITC alone, [dendrimer/albumin-FITC] or [dendrimer-PADRE (PDD)/albumin-FITC]. Twenty four hours post incubation in 37°C/CO₂ incubator, cells with each treatment were analyzed in flow cytometry and gated for human B cells using anti-CD 19-APC. A ratio of 1:10 (w:w) of albumin-FITC and PDD or dendrimer was used. The high levels of delivery (73%) of PDD/albumin-FITC in human B cells, clearly shows that the platform maybe used with protein / polypeptide or their like antigens. FIG. 16 is a series of flow cytometry histograms showing *in vitro* transfection of human B cells (CD19), upper panel, and mice splenocytes population, lower panel, with PDD (PADRE-dendrimer(PDD)/GFP-Plasmid ). Upper panel-Purified human B cells were co-cultured with either of GFP plasmid alone, [dendrimer / GFP plasmid] or [dendrimer-PADRE (PDD) / GFP plasmid] at indicated P:N ratios. Twenty four hours post incubation in 37°C/CO₂ incubator, cells with each treatment were analyzed in flow cytometry for the expression of GFP protein. The high levels of delivery (77%) of PDD/ GFP plasmid in human B cells, clearly shows that the platform efficiently delivers plasmids into B cells and results in the expression of encoded protein / antigen. The lower panel shows flow cytometry Dot Plot diagram when similar experiments were performed with splenocytes of C57BL naive mice and similarly shows the GFP transfection of CD-19 positive cells (B cells). FIG. 17 is a graph showing generation of APC expressing antigen. Six to eight weeks old Female C57BL mice, in groups of five, were immunized twice with OVA protein in TiterMax (Sigma). Ten days post last immunization, the splenocytes of immunized mice were collected and plated at 1 million cells per well in four wells of a 24-well plate in RPMI with 10% FBS, the wells were labeled as "media alone", "PADRE-dendrimer (PDD) alone", "PADRE-dendrimer(PDD)/control-plasmid", and "PADRE-dendrimer(PDD)/OVA-plasmid". Five microgram of plasmids complexed with PADRE-dendrimer (in 1:10 ratio) was added to appropriate wells (target cells). The morning after, each treated / transfected cells were added to untreated splenocytes of same mouse in separate wells. Twenty four hours after stimulation, the levels of INF-γ were detected using ELISA (Thermo) in the supernatants. The levels of IFN-were significantly (P value < 0.006) higher in wells that contained splenocytes treated with [PDD/OVA-plasmid] than all controls which shows the kit may be used for evaluation of T cell responses upon vaccination. The induction of T cell responses were verified by challenge experiments using 50,000 B16-OVA as well as by OVA peptide stimulation (not shown).

### Example 6 - Preparation of Universal DR Binding Peptide-dendrimers For Use In Assessing Efficacy of a Vaccine and Assessing Immunogenicity of A Drug or Biologic

The universal DR binding peptides (including those mentioned in Table 1) can be purchased from any commercial source or synthesized, but generally are purchased from a commercial supplier with a minimum purity of 95%. Standard methods are used for the attachment of the peptide to amino-terminated dendrimers. Attachment of the PADRE peptide to amino-terminated dendrimers, for example, is investigated using two synthetic routes. The amino terminus of the peptide epitope is be protected by acetylation. One route uses the carboxylic acid of the terminal cysteine residue to achieve attachment via standard amidation chemistry. The second route takes advantage of the peptide cysteine's thiol (if the peptide does not have this amino acid it will be added) to react it with bromide groups added to the dendrimer surface by previous treatment with bromocaproyl chloride. Both routes allow the functionalization of dendrimers with peptide epitopes, but the second route provides a 5-methylene spacer between the dendrimer surface and the epitope. Different numbers of epitopes have been attached per dendrimer. An average of two to six epitopes per dendrimer enhances the targeting property of the DNA delivery agents. However, it leaves a large number of unreacted amine groups so that the dendrimer will acquire a large positive charge via protonation at physiological pH values. Characterization of the peptide-derivatized dendrimers was done by UV-Visible and fluorescence spectroscopy, elemental analysis, and MALDI-TOF mass spectrometry. The ratio of peptide-dendrimer to DNA was optimized in order to favor the presentation of the epitope on the surface of the peptide-dendrimer as well as to allow the DNA complex formation. The optimal ratios for efficient gene delivery and presentation of antigen encoded by DNA, using human PBMCS, were determined to be the charged ratios of platform to plasmid of 5:1, 10:1, 20:1.

### Example 7- Simple and Rapid Methods for Immunoevaluation

Transfections are performed by the addition of a first portion or sample of PBMCs to a mixture of plasmid(s) that contain(s) the target gene (i.e., gene encoding the antigen of interest) complexed with a peptide-derivatized-dendrimer (e.g., a G5 dendrimer). The resulting transfected APCs and PBMCs are frozen in DMSO and are used as autologous APCs when co-cultured with a second sample or portion of PBMCs from the same individual. One portion of the PBMCs of each individual will be incubated overnight with a complex made of nanoparticle plus plasmid containing the gene encoding the antigen of interest. The next day, PDD/antigen treated PBMCs (target cells - PBMCs when treated with PDD/plasmid or antigen become APCs expressing antigen) that express the antigen in the context of the individual's MHC are co-cultured with the second sample of PBMCs (effector cells) from the individual.

Universal DR binding peptide(s) on the nanoparticle complex serve as a ligand for MHC class II molecules present on APCs. Described herein is a novel approach of using universal DR binding peptide(s) for their ability to bind to MHC class II as a homing marker for APCs. The transfected APCs that serve as target cells are typically treated with mitomycin C. This mitomycin C treatment upon transfection of APCs with vaccine antigen results in the elimination of their proliferation, and reduction of cytokine expression, thereby resulting in the elimination of interference of target APCs as well as the reduction of possible PADRE-induced background in CTL assays.

### Example 8 ― Generation of APCs Presenting Antigen Encoded by RNA Conjugated to Dendrimers

FIGS. 11 and 12 show results from experiments involving Influenza hemagglutinin (HA) SFERFEIFPKEC (SEQ ID NO:28) T helper epitope decorated dendrimer (DRHA), splenocytes of syngeneic mice (target cells), MHC class 1-HA reacting CD8+T cells of syngeneic mice (effector cells). In the experiment, a mixture of mRNA (including hemagglutininm RNA) derived from hemagglutinin expressing tumors was mixed with DRHA and then added to T cells recognizing the MHC class I restricted hemagglutinin peptide. IFN gamma was measured. The 1FIG. 12 and 13 show that only DRHA was able to induce appreciable level of IFN gamma. Since the hemagglutinin specific CD8+ T cells can recognize the hemagglutinin only if the protein if transplated, processed and complexed with the MHC class I, but they cannot recognize the MHC class II restricted HA peptide conjugated on the dendrimer or exposed on MHC classII molecules, these results clearly show that MHC class II restricted peptide conjugated dendrimers can efficiently transfect APCs with mRNAs. These mRNAs including the antigen of interest are translated into protein, processed and exposed on MHCs molecules. This experiment strongly suggests that an immune response against any tumor associated antigen can be detected by the use of APCs transfected by peptide dendrimers loaded with a mixture of RNA containing also the mRNA of interest. This is particularly important when an immune response from unknown tumor associated antigens expressed by a tumor need to be evaluated.

### Example 9 ― Preparation of Additional Universal DR Binding Peptide-Dendrimers

A dendrimer is decorated with two MHC class II binding peptides, each covering a large number of MHC alleles. Alternatively, to have a less complicated synthesis option, the two sets of dendrimers are made each with one of these peptides and they are mixed 1:1 at point of use.

A first platform is composed of dendrimer decorated with peptide FNNFTVSFWLRVPKVSASHLE (SEQ ID NO:30), and conjugation of the dendrimer with the peptide FNNFTVSFWLRVPKVSASHLE (SEQ ID NO:30). An average of 2 peptides are on each dendrimer. This peptide-derivatized dendrimer is referred to as "FNN-DR." This universal MHC binding peptide is reported to bind to non-human primates, Balb/c, C57B1 as well as the following alleles in humans: DRB1^{∗}1101, DRB1^{∗}1104, HLA-DPB1^{∗}0402, HLA-DRB1^{∗}1101, and HLA-DPB1^{∗}0401.

A second platform is composed of dendrimer decorated with peptide SSVFNVVNSSIGLIM (SEQ ID NO:29), and conjugation of the dendrimer with the peptide SSVFNVVNSSIGLIM (SEQ ID NO:29). An average of 2 peptides are on each dendrimer. This peptide-derivatized dendrimer is referred to as "SSV-DR." This universal DR binding peptide binds to the following alleles: DRB1^{∗}0401 (15%), DRB1^{∗}0405, DRB1^{∗}1101, DRB1^{∗}1302, DRB1^{∗}0701, DRB1^{∗}0802, DRB1^{∗}0901, DRB1^{∗}1501, DRB1^{∗}0101 (24%), and DRB5^{∗}0101.

### Example 10 - Supermotif-Dendrimer Platform For Targeting APCs

Supermotifs target MHC class II and unlike universal T helper epitopes, Class II-associated invariant chain peptides (CLIPs) have no T helper activity, thus making them a good candidate for universal targeting of MHC class II, in particular for immunomonitory applications, and delivery where immunoenhancing is not desired. Dendrimers conjugated to CLIPs can also be used to deliver, for example, FoxP3 siRNA or plasmid(s) to inhibit or induce T regulator cells (T_{Reg} cells) proliferation or activity, as T_{reg} cells express MHC class II. This platform may be used to target APCs for i) specific delivery of anti-microbial or anti-parasitic drugs to macrophages, ii) delivery of cell-specific siRNA or DNA into T_{reg} cells, and iii) to make a T cell immunomonitory kit (requiring less or no activation). The platform can be used, for example, to target drugs (e.g., anti-leishmania drugs) for preventing or treating infection by a parasite (e.g., leishmania) or pathogenic microbe that lives in macrophages and/or dendritic cells. By using the platform, the dose and toxicity of such a drug can be lowered.

### Example 11 ― Measuring T and B Cell Responses For Assessing the Total Immune Response To A Vaccine or Other Intervention

Proper *in vitro* presentation of antigen to PBMCs can also reveal important information on B cell responses that are useful in predicting or assessing the overall immune response against a vaccine, in particular, in cases such as influenza where a rapid B cell response is extremely desired. Such responses may be measured via measurement of vaccine antigen-induced B cell IgG or induction of the enzyme AID (activation induced cytidine deaminase) using the PDD/PBMCs described herein co-cultured with PBMCs of the same individual with the same protocol described for T cell responses above, except the cytokine or other markers/analytes measured are B cell-specific.

## Claims

1. A kit for assessing efficacy of a vaccine, the kit comprising:
a) a plurality of charged highly branched polymeric dendrimers each having conjugated thereto at least one Pan-HLA-DR-binding epitope (PADRE) and at least one agent selected from the group consisting of: peptide or polypeptide antigen, nucleic acid encoding the at least one antigen, negative control polypeptide, and nucleic acid encoding a control polypeptide, wherein the at least one PADRE and the at least one agent are conjugated to the exterior surface of the plurality of charged highly branched polymeric dendrimers such that the at least one PADRE specifically binds to professional antigen presenting cells;
b) at least one buffer;
c) a solid substrate; and
d) instructions for use.

## Patentansprüche

1. Kit zur Prüfung der Wirksamkeit eines Impfstoffs, wobei das Kit aufweist:
a) eine Mehrzahl geladener, hochgradig verzweigter polymerer Dendrimere, wobei an jedes mindestens ein Pan-HLA-DR-bindendes Epitop (PADRE) und mindestens ein Agens, das ausgewählt ist aus der Gruppe, die aus einem Peptid- oder Polypeptid-Antigen, einer Nukleinsäure, die das mindestens eine Antigen kodiert, einem Negativkontrolle-Polypeptid und einer Nukleinsäure, die ein Kontroll-Polypeptid kodiert, besteht, konjugiert sind, wobei das mindestens eine PADRE und das mindestens eine Agens dergestalt an die Außenoberfläche der Mehrzahl der geladenen, hochgradig verzweigten polymeren Dendrimere konjugiert sind, dass das mindestens eine PADRE spezifisch an professionelle antigenpräsentierende Zellen bindet;
b) mindestens einen Puffer;
c) ein festes Substrat; und
d) Gebrauchshinweise.

## Revendications

1. Kit pour évaluer l'efficacité d'un vaccin, ledit kit comprenant :
a) une pluralité de dendrimères polymériques chargés hautement ramifiés, chacun d'entre eux étant conjugué à :
au moins un épitope de liaison Pan-HLA-DR (PADRE) et au moins un agent choisi dans le groupe constitué par: un antigène peptidique ou polypeptidique, un acide nucléique codant pour le au moins un antigène, un polypeptide de contrôle négatif, et un acide nucléique codant pour un polypeptide de contrôle, dans lequel le au moins PADRE et le au moins agent sont conjugués à la surface extérieure de la pluralité de dendrimères polymériques chargés hautement ramifiés de telle sorte que le au moins PADRE se lie spécifiquement aux cellules présentatrices d'antigènes professionnelles ;
b) au moins un tampon ;
c) un substrat solide ; et
d) des instructions d'utilisation.
